# EUROPEAN PATENT SPECIFICATION

(11) **EP 3 334 273 B1**
(45) Date of publication and mention of the grant of the patent: **12.02.2025**
(21) Application number: 16753890.9
(22) Date of filing: 12.08.2016
(51) Int. Cl.: A01H 1/04, C12Q 1/6895, A01H 1/00

(54) **RESISTANCE TO AUSTRALIAN VARIANT OF A. CANDIDA RACE 9 IN BROCCOLI**
RESISTENZ GEGEN DIE AUSTRALISCHE VARIANTE EINER CANDIDA RASSE 9 IN BROKKOLI
RÉSISTANCE À UNE VARIANTE AUSTRALIENNE DE A. CANDIDA RACE 9 DANS LES BROCOLIS

(30) Priority: 12.08.2015 EP 15306288; 11.11.2015 AU 2015255211
(43) Date of publication of application: 20.06.2018
(73) Proprietor: Vilmorin & Cie, 75001 Paris (FR)
(72) Inventor: BERTHY, Pierrick, 26800 Portes lès Valence (FR); DAVID, Perrine, 26800 Portes lès Valence (FR); HURRIER, Pierre, 26800 Portes lès Valence (FR); MARANDEL, Grégoire, 26800 Portes lès Valence (FR)
(74) Representative: Ernest Gutmann - Yves Plasseraud S.A.S.
(86) International application number: PCT/EP2016/069241
(87) International publication number: WO 2017/025627

(56) References cited:
- WO-A1-2010/135782
- PETKOWSKI ET AL: "RACES OF ALBUGO CANDIDA CAUSING WHITE BLISTER RUST ON BRASSICA VEGETABLES IN AUSTRALIA", ISHS ACTA HORTICULTURAE 867: V INTERNATIONAL SYMPOSIUM ON BRASSICAS AND XVI INTERNATIONAL CRUCIFER GENETICS WORKSHOP, BRASSICA 2008,, vol. 867, 1 January 2008 (2008-01-01), pages 133 - 141, XP009187806
- KAUR P AND SIVASITHAMPARAM K: "Host Range and Phylogenetic Relationships of Albugo candida from Cruciferous Hosts in Western Australia, with Special Reference to Brassica juncea", 13 February 2011 (2011-02-13), pages 712 - 718, XP093196727, Retrieved from the Internet <URL:https://apsjournals.apsnet.org/doi/epdf/10.1094/PDIS-10-10-0765>
- E. J. MINCHINTON ET AL: "Evaluation of the efficacy and economics of irrigation management, plant resistance and Brassicaspot(TM) models for management of white blister on Brassica crops", AUSTRALASIAN PLANT PATHOLOGY, vol. 42, no. 2, 7 December 2012 (2012-12-07), AU, pages 169 - 178, XP055238220, ISSN: 0815-3191, DOI: 10.1007/s13313-012-0181-z
- SANTOS M R ET AL: "Evaluation of a core collection of Brassica oleracea accessions for resistance to white rust of crucifers (Albugo candida) at the cotyledon stage", GENETIC RESOURCES AND CROP EVOLUTION, KLUWER ACADEMIC PUBLISHERS, DO, vol. 51, no. 7, 1 November 2004 (2004-11-01), pages 713 - 722, XP019243221, ISSN: 1573-5109, DOI: 10.1023/B:GRES.0000034577.82868.5D
- KOLE C ET AL: "Molecular mapping of a locus controlling resistance to Albugo candida in Brassica rapa", PHYTOPATHOLOGY, AMERICAN PHYTOPATHOLOGICAL SOCIETY, US, vol. 86, no. 4, 1 January 1996 (1996-01-01), pages 367 - 369, XP002566419, ISSN: 0031-949X, DOI: 10.1094/PHYTO-86-367
- PETKOWSKI J E ET AL: "Molecular phylogenetic relationships between Albugo candida collections on the Brassicaceae in Australia", PLANT PATHOLOGY (OXFORD), vol. 59, no. 2, April 2010 (2010-04-01), pages 282 - 288, XP002752796, ISSN: 0032-0862

## Description

The present invention relates to resistance in plants of *Brassica oleracea* to white blister rust caused by the oomycete *Albugo candida* race 9, especially to the Australian variant of *Albugo candida* race 9. According to the invention, the resistance is provided by DNA sequences, introgressed in the genome of a *Brassica oleracea* plant, on chromosome 4. The introgressed sequences can be present homozygously or heterozygously in the genome of a *Brassica oleracea* plant. A particularly preferred resistant plant is broccoli. The present invention further relates to a method for providing *B. oleracea* plants which are resistant to white blister rust, DNA markers for white blister rust resistance and their use in identifying plants resistant to white blister rust caused by the oomycete *Albugo candida* race 9, especially to the Australian variant.

### Background:

The oomycete *Albugo candida* (Pers. ex. Lev.) Kuntze is an obligate biotrophic pathogen of the Brassicaceae family, also known as the crucifer family, and causes the widespread disease known as white rust (also known as white blister, white blister rust, staghead, *A. cruciferum* or *A. cruciferatum*)*.* This disease is destructive to many vegetable and oilseed crops such as broccoli, cabbage, rape, mustard and radish.

White rust has been observed worldwide, in countries as geographically and environmentally diverse as India, Canada, Europe and Australia. In Australia, white blister rust has spread to Brassicaceae crops in southern parts of the country.

*A. candida* is highly specialized, grows between living host cells and causes a range of symptoms that can be the result of local or systemic infection. The genus *Albugo* includes several species that cause white rust on a range of hosts and within *A. candida* various host-specialized forms have been reported. One of the characteristics of *A. candida* is thus its high degree of host specificity.

The growth of *A. candida* leads to two types of infections, local or systemic. Local infection is characterised by white or creamy-yellow pustules of zoosporangia that form under the epidermis of the host. These usually develop on the lower (abaxial) surface of the leaf and to a lesser extent on the upper (adaxial) surface and occur more commonly on mature leaves. However, they may be localized on any aerial host organ. Initially, the pustules are small and discrete but eventually become large and confluent. Once the pustule is fully developed, the host epidermis ruptures to release a dry, powdery burst of zoosporangia. Subsequently, necrosis of the surrounding leaf tissue may occur.

Localized infection does not usually result in extensive yield loss but would still lead to unmarketable products. Systemic infection can have a severe impact on the productivity of crops grown for seed or floral parts. Systemic infection appears to trigger sexual reproduction of the oomycete and causes distortion, hyperplasia and hypertrophy of the inflorescences, stems and leaves. It can also result in sterility if the flower petals, ovules and pollen grains are malformed as a result of the disease.

*A. candida* grows best at moderate temperatures of between 10 and 20°C and in moist conditions. A leaf wetness period of 2.5 hours is sufficient to result in infection, which becomes symptomatic after an incubation period of 10 to 14 days.

Brassica is a plant genus in the family Brassicaceae (formerly referred to as Cruciferae). The genus Brassica comprises a number of important agricultural and horticultural crops, including rape, cauliflower, broccoli and turnip. Almost all parts of these plants can be used as food. Rape and rape seed are also used for oil, both for consumption and for fuel. Some species with white or purple flowers or distinct colour or shaped leaves are cultivated for ornamental purposes. The Brassicaceae family occurs worldwide and comprises annuals, biennials and perennials. The family also comprises a large number of wild species.

In some crops white rust can cause huge losses in yield. In other Brassica crops white rust affects the appearance of the plant such that the crop is no longer commercially viable because of the cosmetic damage. There is therefore a great need for Brassica crops which are resistant to white rust.

The inheritance of resistance to white blister rust has been shown to vary both between and within host species. Resistance to white rust has been studied in *B. rapa, B. napus* and *A*. *thaliana* and has been found to be controlled either by a single dominant gene, as in B. *juncea, B. carinata, B. nigra,* or by multiple genes where the resistance is said to be polygenic. Resistance to white rust has also been associated with specific biochemical properties of the host, such as elevated levels of certain sugars, chlorophyll or phenols.

*A. candida* is now subdivided into several races that are specialised to infect specific hosts. The current classification of *A. candida* biological races is based on the response of Brassicaceae hosts to infection by various isolates of *A. candida.* The different races identified to date are reported in table 1. It is to be noted that for *A. candida,* the term "race" as used herein refers to a variety of a pathogen that can infect some species of a host genus and not others whereas in most pathosystems it is taken to mean a variety of pathogen that can infect some varieties of a host species but not others.

**Table 1:**

| Race | Host Scientific Name | Host Common Name |
|---|---|---|
| 1 | *Raphanus sativus L.* | Radish |
| 2 | *Brassica juncea (L) Coss* | Indian Mustard |
| 3 | *Armoracia rusticona Gaertn., Mey., & Schreb.* | Horseradish |
| 4 | *Capsella bursa-pastoris (L) Medik.* | Sherpherd's Pulse |
| 5 | *Sisybrium officinale (L) Scop* | Hedge/Tumble mustard |
| 6 | *Rorippa islandica (L) Bess.* | Marsh/Yellow Water Cress |
| 7 | *Brassica rapa L (campestris, pekinensis, chinensis*) | Chinese Mustard, Chinese Cabbage, Pak Choi, Field and Black Mustard |
| 8 | *Brassica nigra (L) Koch* | Black Mustard |
| 9 | *Brassica oleracea L* | Broccoli, Brussels Sprouts, Cauliflower, Kale, Kohrabi, Cabbage |
| 10 | *Brassica kaber (DC) Wheeler (Sinapsis arvensis)* | Charlock |
| 11 | *Brassica carinata* | Ethiopian Mustard |
| 12 | *Brassica juncea* | Indian Mustard |
| 13 | *Brassica campestris* var. toria | Indian Rape |
| 14 | *Brassica juncea* | Indian Mustard |
| 15 | *Brassica juncea* | Indian Mustard |
| 16 | *Brassica juncea* | Indian Mustard |
| 17 | *Brassica juncea* | Indian Mustard |

In the last years, these races of *A. candida* have been subdivided to acknowledge the existence of different variants within a race. Two broad variants of race 7 have been identified, based on their virulence to two varieties of *B*. *rapa,* and four variants of race 2 have been characterised, based on their virulence to eleven cultivars of *B. juncea.*

*A. candida* race 9 has spread to *B. oleracea* var. *italica* (broccoli) and B. *oleracea* var. *botrytis* (cauliflower) crops in southwest Australia, making this pathogen the number one concern for broccoli and cauliflower breeding in Australia. This oomycete pathogen is growing on Broccoli leaves, stem or curds showing white spot symptoms on leaves and transforming buds into white erected pin heads that affect the curd quality and shelf life. The Australian *A. candida* strain infecting B. *oleracea* differs from the *A. candida* race 9 generally encountered in other parts of the world, and referred to as European variant; this European variant causes disease essentially on cabbage.

Indeed, the Australian race 9 is in some experiments more aggressive towards broccoli than towards cabbage while in Europe, the race 9 appears to be more aggressive towards cabbage and Brussels sprouts. It is therefore reported that race 9 comprises at least two variants, the European variant, more aggressive towards cabbage and the Australian variant more aggressive towards broccoli (Petkowski et al, Proc. Vth IS on Brassica & XVIth Crucifer Genetics WS: 133-141; 2010); for example *Iron* CMS and *Belstar* commercial varieties show resistance to the European variant but are susceptible to the Australian variant, as reported in WO2010/135782.

The white rust outbreaks caused by *A. candida* in many broccoli crops have been reported to be very serious. In many situations infection rates have been as high as 100%, with the only recourse being to destroy the entire crop. In neglected fields, complete infection of every part of every plant, from the leaves to the broccoli head, is often observed. The spore load from the mature sporangia is so extensive that a cloud of spores can be seen upon physical disruption of an infected plant.

At the moment few agents are known which can be used to control white rust in Brassicas. Current control methods generally involve a combination of management practices (controlled watering, ventilation, balanced program of nutrition) and a fungicide spray program. However, an increasing number of countries have implemented policy aimed at reducing the use of chemical crop protection agents. If the use of chemical control agents is banned completely, this would result in major problems in the cultivation of Brassica species.

Genetic resistance to *A. candida* in broccoli (*Brassica oleracea* var. *italica*) would be beneficial in the control of white blister rust. Such resistance would not only increase the stability of crop protection, but would also result in a reduced, or eliminated, requirement for environmentally harmful, and not always effective, fungicide applications. Such resistance could also be beneficial to other *B. oleracea* plants such as cauliflower, Brussel sprouts, white cabbage, savoy cabbage, etc.

WO2008133503 illustrates *B. oleracea* plants allegedly containing a monogenic dominant resistance gene to *A. candida,* but not to the Australian variant of *A. candida* race 9. WO2011036108 illustrates *B. oleracea* plants allegedly containing a monogenic semi-dominant resistance gene to *A. candida,* present on chromosome 2 but not conferring resistance to the Australian variant.

WO2010135782 illustrates *B. oleracea* plants containing a monogenic dominant resistance gene to *A. candida* located on chromosome 1. This gene has been able to confer resistance to some strains of the Australian variant of *A. candida* race 9 but appears to be unable to generally confer resistance to white rust caused by *A. candida* race 9 Australian variant, irrespective of the strain. Similarly, the variety *Tyson* (Minchinton et al, Australian Plant Pathol 2013; 42:169-178), although it is disclosed as resistant to the Australian variant of *A. candida* race 9, appears to be unable to generally confer resistance to white rust caused by *A. candida* race 9 Australian variant, irrespective of the strain, as illustrated in the examples (see Ex. 6 and 7).

Therefore, there is still an unfulfilled need for broccoli plants with a resistance to the Australian variant of *A. candida* race 9, namely a general resistance which is not restricted to some strains.

### SUMMARY

The present inventors have identified a resistance to the Australian variant of the *A. candida* race 9 pathogen and have been able to introgress this resistance into *B. oleracea* plants, thus obtaining resistant *B. oleracea* plants, more specifically broccoli resistant plants. The resistance of the present invention is imparted by the newly discovered sequences conferring the resistance, said resistance being of monogenic and dominant nature and transferable to different *B. oleracea* genetic backgrounds.

The present invention provides introgressed sequences conferring, when present in homozygous or in heterozygous state, the phenotype of resistance to the Australian variant of the *A. candida* race 9. The invention also provides *B. oleracea* plants that display resistance to Australian variant of the *A. candida* race 9, namely broccoli plants, as well as methods that produce or identify *B. oleracea* var. *italica* broccoli plants that display resistance to Australian variant of the *A. candida* race 9 as well as seeds, roots and other plant parts such as pollen and ovules containing the introgressed sequences conferring the resistance. The invention also discloses molecular genetic markers, especially SNPs, linked to the introgressed sequences conferring the resistance, i.e. linked to the resistance locus which is of dominant nature.

### Definitions:

The ***B. oleracea*** plant resistant to white rust disease caused by the oomycete *A. candida* race 9 Australian variant plant may be selected from the group consisting of *B. oleracea* convar. Botrytis var. italica (broccoli) or *B. oleracea* convar. botrytis var. botrytis (cauliflower) and most preferably is broccoli.

An ***A. candida* race 9 Australian variant** is a strain of *A. candida* corresponding to the race 9 according to the current classification of *A. candida* biological races established by Pound and William in 1963 (Pound and William, 1963, Phytopathology 53:1146-1149), which is further characterized as being more aggressive towards broccoli than towards cabbage by opposition to the European variant race 9 which is more aggressive towards cabbage and Brussels sprouts. The Australian variant is also known as being the variant responsible for the white blister rust outbreaks in Victorian broccoli and cauliflower crops in the summer 2001-2002 and the subsequent spread of disease to all Australian broccoli and cauliflower cropping areas (Petkowski et al, Proc. Vth IS on Brassica & XVIth Crucifer Genetics WS: 133-141).

Different strains of *A. candida* race 9 Australian variant co-exist, *inter alia* during field infection, which may have different virulence on the infected plants.

The term **"Resistance"** is as defined by the ISF (International Seed Federation) Vegetable and Ornamental Crops Section for describing the reaction of plants to pests or pathogens, and abiotic stresses for the Vegetable Seed Industry.

Specifically, by resistance, it is meant the ability of a plant variety to restrict the growth and development of a specified pest or pathogen and/or the damage they cause when compared to susceptible plant varieties under similar environmental conditions and pest or pathogen pressure. Resistant varieties may exhibit some disease symptoms or damage under heavy pest or pathogen pressure.

**Susceptibility:** The inability of a plant variety to restrict the growth and development of a specified pest or pathogen.

A *B*. *olearacea* plant susceptible to white rust, especially caused by *A. candida* race 9 Australian variant, is for example the commercially available plant 'Belstar' or 'Fiesta' from Bejo, the variety 'Rumba' from HM. Clause, the varieties 'Patriot' and 'Green Belt' from Sakata or the variety 'Monaco' from Syngenta. All the commercially available varieties of *B*. *olearacea* broccoli are, to date, susceptible to white rust caused by *A. candida* race 9 Australian variant, apart *Booster* variety of HM. Clause SA and *Tyson* variety of Syngenta, which are however not resistant to all infections by *A. candida* race 9 Australian variant.

A plant according to the invention has thus at least improved resistance to *A. candida* race 9 Australian variant, and especially with respect to the broccoli plant *Booster,* and more generally with respect to any commercial variety of broccoli.

As used herein, the term **"offspring"** or **"progeny"** refers to any plant resulting as progeny from a vegetative or sexual reproduction from one or more parent plants or descendants thereof. For instance an offspring plant may be obtained by cloning or selfing of a parent plant or by crossing two parents plants and include selfings as well as the F1 or F2 or still further generations. An F1 is a first-generation offspring produced from parents at least one of which is used for the first time as donor of a trait, while offspring of second generation (F2) or subsequent generations (F3, F4, etc.) are specimens produced from selfings of F1's, F2's etc. An F1 may thus be (and usually is) a hybrid resulting from a cross between two true breeding parents (true-breeding is homozygous for a trait), while an F2 may be (and usually is) an offspring resulting from self-pollination of said F1 hybrids.

As used herein, the term **"cross", "crossing", "cross pollination" or "cross-breeding"** refer to the process by which the pollen of one flower on one plant is applied (artificially or naturally) to the ovule (stigma) of a flower on another plant.

As used herein, the term **"gene"** refers to any segment of DNA associated with a biological function. Thus, genes include, but are not limited to, coding sequences and/or the regulatory sequences required for their expression. Genes can also include nonexpressed DNA segments that, for example, form recognition sequences for other proteins. Genes can be obtained from a variety of sources, including cloning from a source of interest or synthesizing from known or predicted sequence information, and may include sequences designed to have desired parameters.

As used herein, the term **"genotype"** refers to the genetic makeup of an individual cell, cell culture, tissue, organism (e.g., a plant), or group of organisms.

As used herein, the term **"heterozygote"** refers to a diploid or polyploid individual cell or plant having different alleles (forms of a given gene or sequences) present at least at one locus.

As used herein, the term **"heterozygous"** refers to the presence of different alleles (forms of a given gene or sequences) at a particular locus.

As used herein, the terms **"homolog"** or **"homologue"** refer to a nucleic acid or peptide sequence which has a common origin and/or functions similarly to a nucleic acid or peptide sequence from another species.

As used herein, the term **"homozygote"** refers to an individual cell or plant having the same alleles at one or more loci on all homologous chromosomes.

As used herein, the term **"homozygous"** refers to the presence of identical alleles at one or more loci in homologous chromosomal segments.

As used herein, the term **"hybrid"** refers to any individual cell, tissue or plant resulting from a cross between parents that differ in one or more genes.

As used herein, the term **"locus"** (plural: "loci") refers to any site that has been defined genetically. A locus may be a gene, or part of a gene, or a DNA sequence, and may be occupied by different sequences. A locus may also be defined by a SNP (Single Nucleotide Polymorphism), or by several SNPs.

By **introgression,** it is meant the infiltration of the genes or of genomic sequences of one species, variant or strain, into the gene pool of another one from an initial hybrid between these species, variants or strains.

### Detailed description of the invention:

The present invention is directed to *B. oleracea* var. *italica* broccoli plants that display resistance to Australian variant of *A. candida* race 9, as well as methods that produce or identify *B. oleracea* broccoli plants that display resistance to infection by Australian variant of *A. candida* race 9. The present invention also discloses molecular genetic markers, especially SNPs, linked to the resistance locus. The inventors have also shown that the sequences responsible for the resistance can be introgressed into different genetic backgrounds and still confer the resistance phenotype (as demonstrated *inter alia* in example 1 with different F2 populations and example 4 with 2 inbred lines).

The seeds and plants according to the invention have been obtained from an initial cross between a Romanesco plant, the introgression partner displaying the phenotype of interest, and an inbred line of broccoli, the recurrent susceptible parent, followed by backcross and a dihaploidization program. A sample of this *Brassica oleracea var italica* (broccoli) BROCCO-C4 seed has been deposited by HM.Clause S.A. Rue Louis Saillant, Z.I. La Motte, 26800 Porte les Valence, France pursuant to, and in satisfaction of, the requirements of the Budapest Treaty on the International Recognition of the Deposit of Microorganisms for the Purposes of Patent Procedure (the "Budapest treaty") with the National Collection of Industrial, Food and Marine Bacteria (NCIMB), (NCIMB Ltd, Ferguson Building, Craibstone Estate, Bucksburn, Aberdeen AB21 9YA, United Kingdom), on 9^{th} July 2015, under accession number NCIMB 42433.

The plants grown from these deposited seeds are broccoli plants resistant to white blister rust caused by any Australian variant of *A. candida* race 9 and are commercially acceptable phenotype.

A deposit of this *Brassica oleracea varitalica -* BROCCO-C4 seed is maintained by HM.Clause S.A. Rue Louis Saillant, Z.I. La Motte, 26800 Porte les Valence, France.

According to a first aspect, the present invention is thus directed to a *B. oleracea* var. *italica* plant or seed, which is resistant to white blister rust caused by the oomycete *Albugo candida* race 9 Australian variant, comprising in its genome introgressed sequences or interval conferring said resistance to white blister rust. Said introgressed sequences confer the resistance to a *B. oleracea* var. *italica* plant or seed when present either homozygously, or heterozygously.

The introgressed interval acts as a single dominant allele of a resistance gene responsible for the phenotype (i.e. the resistance trait is monogenic and dominant). Plants homozygous and heterozygous for the introgressed interval both fully exhibit the white rust resistance phenotype. This phenotype can be used to identify progeny that bear the claimed introgressed sequences or interval. The introgressed interval acting as a resistance gene confers the phenotype of interest and is unexpectedly unlinked to negative features incompatible with marketability of the plants, seed or head, such as presence of leaflets on the petioles or "cat-eyes", i.e. the presence of some beads which prematurely break into yellow flowers.

The resistance to *A. candida* race 9 Australian variant infection is advantageously determined by comparison to a susceptible (commercial) line, for example Belstar, Fiesta or Rumba, or to a line resistant to only some strains, for example Booster or Tyson. The resistance may be determined by the pathological test described in example 1, preferably 15 days after inoculation. In this resistance scoring, a score of "1" corresponds to leaves with more than 75% of the surface with sporulation, a score of "3" corresponds to between 51 and 75% of the surface with sporulation, a score of "5" is 26 to 40% of the surface with sporulation, a score of "7" is 13 to 25%, a score of "8" is 1 to 12%, and "9" corresponds to the absence of sporulation on the leaves. A plant having a score of 9, i.e. without sporulation on the leaf surface, is to be considered as resistant, and more specifically highly resistant.

The introgressed sequences are preferably to be found on chromosome 4 in the *B. oleracea* genome and thus confer resistance to white blister rust caused by the oomycete *A. candida* race 9 Australian variant when they are present on either one or both homologous chromosomes 4. The introgressed sequences conferring the resistance are more preferably located within the chromosomal region of chromosome 4 which is delimited on one side by the SNP BO-0108436 (SEQ ID No:5) and on the other side by the SNP BN-0067793 (SEQ ID No:15), and preferably between SNP BO-0108436 (SEQ ID No:5) and SNP BO-0108751 (SEQ ID No:11).

The specific polymorphisms corresponding to the SNPs (Single Nucleotide Polymorphism) referred to in this description, as well as the flanking sequences of these SNPs in the *B. oleracea* genome, are given in the experimental section (see *inter alia* table 11) and accompanying sequence listing. Their location with respect to the version V2.1 of the *Brassica oleracea* TO1000 genome, on chromosome 4, is indicated in table 11, and their flanking sequences are also illustrated in table 11.

The introgressed sequences or interval conferring the resistance are preferably chosen from the introgressed sequences present in the genome of a plant of *B. oleracea* var. Italica -BROCCO-C4, representative seeds of which are deposited at the NCIMB under the accession number NCIMB 42433. They are especially chosen from the introgressed sequences present on chromosome 4 of said *B. oleracea* var. Italica -BROCCO-C4. Indeed, the deposited seeds comprise, on one chromosome 4 homologue, introgressed sequences conferring the phenotype of interest, wherein said introgressed sequences are also conferring the phenotype in *B.* oleracea genetic background. A sample of this *B. oleracea* var. Italica -BROCCO-C4 seed has been deposited with the National Collection of Industrial, Food and Marine Bacteria (NCIMB), (NCIMB Ltd, Ferguson Building, Craibstone Estate, Bucksburn, Aberdeen AB21 9YA, United Kingdom), on 9^{th} July 2015, under accession number NCIMB 42433. The deposited seeds are hybrid seeds, they bear the introgression sequences, conferring the resistance, on only one chromosome 4 homologue. The resistance sequences can be easily identified *inter alia* by the markers disclosed in the application.

The sequences conferring the resistance are present in the genome of all deposited seeds; as such the genome of these deposited seeds thus represent a reservoir of introgressed sequences in the *B. oleracea* genome conferring resistance to white rust caused by *A. candida* race 9 Australian variant according to the invention. A plant or seed of the invention comprises in its genome introgressed sequences which are chosen from this reservoir.

Whereas all deposited seeds possess an introgressed fragment at the same locus on one chromosome 4, and conferring the phenotype according to the invention, this introgressed fragment may slightly vary in length between the seeds.

The present invention is thus also directed to a *B. oleracea* var. *italica* seed or plant, preferably a cultivated *B. oleracea* var. *italica* broccoli seed or plant, having in its genome introgressed sequences conferring resistance to white rust caused by *A. candida* race 9 Australian variant, wherein said introgressed sequences conferring the resistance are chosen from the introgressed sequences present in the genome of a seed of *B. oleracea* var. Italica -BROCCO-C4 corresponding to NCIMB 42433 deposit.

By "introgressed sequences or intervals at a given locus" or "introgressed sequences or intervals present/found at a given locus", it is to be understood that the genomic interval found at this given locus has not the same sequence as the corresponding genomic interval found in the B. oleracea broccoli germplasm before introgression, but has, at this locus, the sequence found in the corresponding genomic interval of *B. oleracea* var. Italica -BROCCO-C4 (NCIMB 42433) at the same locus; the introgressed sequences are thus exogenous with respect to a plant of the invention. By having the "same sequence", it means that the two sequences to be compared are identical to the exception of potential point mutations which may occur during transmission of the genomic interval to progeny, i.e. preferably at least 99% identical on a length of 1 kbase. It can be deduced that a genomic interval under test has the same sequence, in the sense of the invention, as the corresponding genomic interval found in the *B. oleracea* var. Italica - BROCCO-C4 at the same locus, if said genomic interval under test is also capable of conferring resistance to white rust caused by *A. candida* race 9 Australian variant.

The presence of introgressed sequences into the genome of a *B. oleracea* plant, seed or cell may for example be shown by GISH (genetic in situ hybridization). GISH is indeed a powerful technique for detection of the introgression of chromatin material from one species or strain onto another species or strain. The advantage of GISH is that the introgression process is visualized by means of 'pictures of the introgressed genome'. With this technique, it is also possible to establish if a particular region of the genome is homozygous or heterozygous, thanks to the use of molecular cytogenetic markers which are co-dominant. By this technique, it is also possible to determine in which chromosome an introgressed gene of interest is present.

According to a preferred embodiment, the introgressed sequences conferring the resistance phenotype of the invention, i.e. resistance to white blister rust caused by the oomycete *Albugo candida* race 9 Australian variant, are introgressed from a romanesco *B. oleracea* plant.

According to a preferred embodiment, the introgressed sequences conferring the resistance, and which are also to be found in the genome of the deposited seeds, are introgressed on chromosome 4 of a *B. oleracea* plant according to the invention, preferably broccoli, they are more precisely within the chromosomal region of chromosome 4 which is delimited on one side by the SNP BO-0108436 (SEQ ID No:5) and on the other side by the SNP BN-0067793 (SEQ ID No:15), and more preferably between SNP BO-0108436 (SEQ ID No:5) and SNP BO-0108751 (SEQ ID No:11). In other words, in the genome of a *B. oleracea* plant or seed of the invention, the section on chromosome 4 within the region delimited on one side by the SNP BO-0108436 (SEQ ID No:5) and on the other side by the SNP BN-0067793 (SEQ ID No:15), comprises sequences which are responsible for the resistance to white rust caused by *A. candida* race 9 Australian variant, in *B. oleracea* genetic background, and more preferably between SNP BO-0108436 (SEQ ID No:5) and SNP BO-0108751 (SEQ ID No:11). Said introgressed sequences are preferably from a romanesco *B. oleracea* plant.

According to a preferred embodiment, the introgressed sequences present in the genome of a plant or seed of the invention are to be found at one or more of the following loci:
- locus encompassing SNP BN-0067786 (SEQ ID No:14), and / or
- locus encompassing SNP BO-0108746 (SEQ ID No:6).

The locus encompassing SNP BO-0108746 is highly preferred.

It is noted in this respect that specific positions in a chromosome can indeed be defined with respect to single nucleotide polymorphism, insofar as the flanking sequences of said SNPs are defined. The present inventors have used SNPs, identified by their flanking sequences, present in all *B. oleracea* genomes, especially *B. oleracea* var. Italica and Romanesco B. *oleracea,* to discriminate between introgressed and endogenously residing sequences and to track down the introgressed sequences from *Romanesco* in the *B. oleracea* broccoli genome.

The present inventors have identified that introgressed sequences essential for the phenotype of interest, i.e. white rust caused by *A. candida* race 9 Australian variant, are to be found in the vicinity of the SNP BN-0067786 (SEQ ID No:14) or preferably in the vicinity of SNP BO-0108746 (SEQ ID No:6), or in the vicinity of both SNPs. Preferably the introgressed sequences are to be found in a locus encompassing the position of SNP BO-0108746. It is to be noted that the locus of SNP BN-0067786 and SNP BO-0108746 are located within the chromosomal region defined above, i.e. delimited on one side by the SNP BO-0108436 and on the other side by the SNP BN-0067793 on *B. oleracea* chromosome 4.

When the introgressed sequences conferring the resistance are found in a locus encompassing SNP BN-0067786 then the allele of SNP BN-0067786 is the allele of SNP BN-0067786 found in the Romanesco introgression partner, and also in the deposited resistant *B. oleracea* var. Italica -BROCCO-C4, and demonstrated in the experimental section as genetically linked to the resistance, i.e. allele T of SNP BN-0067786. The 5' flanking region of SNP BN-0067786, or the 3' flanking region of SNP BN-0067786, or both regions, are also identical *B. oleracea* var. Italica -BROCCO-C4 sequences in this region. Therefore, the SNP BN-0067786 may form part of the 3' border or 5' border of the introgressed interval, or may be within the introgressed interval conferring the desired phenotype.

When the introgressed sequences conferring the resistance are found in a locus encompassing SNP BO-0108746, then the allele of SNP BO-0108746 is the allele of SNP BO-0108746 found in the Romanesco introgression partner, and also in the deposited resistant BROCCO-C4, and demonstrated in the experimental section as genetically linked to the resistance i.e. allele C of SNP BO-0108746. The 5' flanking region of SNP BO-0108746, or the 3' flanking region of SNP BO-0108746, or both regions, are also identical to *B. oleracea* var. Italica -BROCCO-C4 sequences in this region. Therefore, the SNP BO-0108746 may form part of the 3' border or 5' border of the introgressed interval, or may be within the introgressed interval conferring the desired phenotype.

The presence of the introgressed sequences of interest can indeed be revealed by the presence of specific alleles of given SNPs, wherein said alleles are characteristic of the introgression partner, thus associated with the resistance, and distinct from the allele of the recurrent B. oleracea broccoli parent for these SNPs, associated with the susceptibility. The alleles of given SNPs can thus reflect the presence of the introgression sequences of the invention. Examples of appropriate SNPs are all or part of the list consisting of SNP BO-0108436 (SEQ ID No:5), SNP BO-0108746 (SEQ ID No:6), SNP BO-0108747 (SEQ ID No:7), SNP BO-0108748 (SEQ ID No:8), SNP BO-0108749 (SEQ ID No:9), SNP BO-0108750 (SEQ ID No:10), SNP BO-0108751 (SEQ ID No:11), SNP BO-0108752 (SEQ ID No:12), SNP BO-0108753 (SEQ ID No:13), SNP BN-0067786 (SEQ ID No:14) and SNP BN-0067793 (SEQ ID No:15). These alleles, associated with the resistance are *inter alia* allele G of SNP BO-0108436 (SEQ ID No:5); allele C of SNP BO-0108746 (SEQ ID No:6); allele G of SNP BO-0108747 (SEQ ID No:7); allele A of SNP BO-0108748 (SEQ ID No:8); allele C of SNP BO-0108749 (SEQ ID No:9); allele A of SNP BO-0108750 (SEQ ID No:10); allele G of SNP BO-0108751 (SEQ ID No:11); allele C of SNP BO-0108752 (SEQ ID No:12); allele C of SNP BO-0108753 (SEQ ID No:13); allele T of SNP BN-0067786 (SEQ ID No:14) and allele G of SNP BN-0067793 (SEQ ID No:15); these alleles correspond to the alleles of these SNPs found in the deposited seeds NCIMB 42433. Table 11 discloses for the 11 SNPs of the invention the allele which is associated with the resistance phenotype, i.e. the allele which co-segregates with the resistance phenotype, and the allele which is associated with the susceptibility.

A plant or seeds of the invention is preferably characterized by allele C of BO-0108746, preferably in combination with at least one, preferably at least two, 3, 5 or all of allele G of BO-0108436, allele G of BO-0108747, allele A of BO-0108748, allele C of BO-0108749, allele A of BO-0108750, allele G of BO-0108751, allele C of BO-0108752, allele C of BO-0108753, allele T of BN-0067786 and allele G of BN-0067793. As disclosed in example 4, allele C of BO-0108746 appears very highly associated with the phenotype and is thus mostly preferred according to the invention.

According to another embodiment, the introgressed sequences present in the genome of a plant or seed of the invention are to be found at one or more of the following loci:
- locus encompassing SNP BO-0108436 (SEQ ID No:5);
- locus encompassing SNP BO-0108747 (SEQ ID No:7);
- locus encompassing SNP BO-0108748 (SEQ ID No:8);
- locus encompassing SNP BO-0108749 (SEQ ID No:9);
- locus encompassing SNP BO-0108750 (SEQ ID No:10);
- locus encompassing SNP BO-0108751 (SEQ ID No:11);
- locus encompassing SNP BO-0108752 (SEQ ID No:12);
- locus encompassing SNP BO-0108753 (SEQ ID No:13) and /or
- locus encompassing SNP BN-0067793 (SEQ ID No:15).

in addition or in place of the introgressed sequences found at the locus encompassing SNP BN-0067786 and/or preferably SNP BO-0108746 according to the previous embodiment, preferably in all these 11 loci.

According to a preferred embodiment, introgressed sequences are to be found at the loci encompassing SNP BO-0108436, SNP BO-0108746, SNP BO-0108747, SNP BO-0108748, SNP BO-0108749, SNP BO-0108750 and SNP BO-0108751.

Consequently, a resistant plant or seed of the invention may also be characterized by the allele T of SNP BN-0067786, or preferably by allele C of SNP BO-0108746, or both. They may also be characterized in addition to allele C of BO-0108746, by at least one of the following alleles: allele G of SNP BO-0108436; allele G of SNP BO-0108747; allele A of SNP BO-0108748; allele C of SNP BO-0108749; allele A of SNP BO-0108750; allele G of SNP BO-0108751; allele C of SNP BO-0108752; allele C of SNP BO-0108753; allele T of SNP BN-0067786 and allele G of SNP BN-0067793.

The presence of the introgressed sequences can also be revealed by genic amplification of sequences in the proximity of the SNPs defined in the present invention, especially SNP BO-0108746 and comparison with the sequence of the respective amplification fragment, obtainable by carrying out the amplification on seeds deposited at the NCIMB under accession number NCIMB 42433. Primers for the genic amplification can be defined by use of the flanking sequences disclosed in the present invention, potentially in combination with the available *B. oleracea* genome assembly.

Preferably, the introgressed sequences conferring the phenotype of interest, are in linkage disequilibrium with the allele of SNP BO-0108746. According to a preferred embodiment, the allele of SNP BO-0108746 is "C". According to another embodiment, the allele 'T' of SNP BN-0067786 and allele C of SNP BO-0108746 are in linkage disequilibrium with the introgressed sequences conferring the resistance. Linkage disequilibrium indeed is used to describe common inheritance of genomic sequences in a cross population analysis when no linkage exists. Linkage describes common inheritance of genomic sequences in a population structure pending on the frequency of recombination.

The linkage disequilibrium score may be any positive score, meaning that the association of SNP BO-0108746 with the introgressed sequences is not random.

In a plant or seed of the invention, thus comprising in its genome introgressed sequences, said introgressed sequences are preferably to be found in the genome at a genetic distance of less than 20 cM, preferably less than 15 cM, most preferably less than 10 cM, and even preferably less than 5 cM from the locus corresponding to SNP BO-0108746.

In another embodiment, the introgressed sequences are to be found in the genome of a plant or seed of the invention in the vicinity of both SNPs BO-0108746 and BN-0067786, preferably at less than 20 cM from both SNPs, or preferably less than 15 cM from both; for example the introgressed sequences are located at less than 5 cM from the locus corresponding to SNP BO-0108746 and at 15 cM or less from SNP BN-0067786.

According to an embodiment, a seed or plant of the invention, preferably a broccoli seed or plant, is characterized by the presence of allele C of SNP BO-0108746 on chromosome 4, optionally in combination with absence of allele A of said SNP. Indeed, presence of allele C of SNP BO-0108746 confirms the presence of introgressed sequences at the locus of SNP BO-0108746 and thus of the resistance; moreover the absence of allele A indicates that the introgressed sequences are homozygously present, i.e. present on all the homologues of chromosome 4. Alternatively, a seed or plant of the invention, preferably a broccoli seed or plant, is characterized by the presence of both alleles C and A of SNP BO-0108746 on chromosome 4, confirming the heterozygous presence of the introgressed sequences.

According to a preferred embodiment of the present invention, the introgressed sequences or interval present in the genome of a broccoli seed or plant and conferring the resistance to white rust caused by *A. candida* race 9 Australian variant, are at least 5 kilobases long, and preferably at least 8, 10 or 15 kb long.

Preferably, the introgressed sequences or intervals are however not too long in order to avoid introgression of non-commercial features associated with Romanesco introgression partner. It is thus preferred according to the invention that the introgressed sequences mentioned above are less than 25 cM in length, preferably less than 20 cM or less than 15 cM. According to more preferred embodiments, the introgressed sequences are less than 10 cM or even less than 5 cM in length and most preferably less than 5 cM in order to avoid or limit linkage drag.

According to a preferred embodiment, said introgressed sequences are minimized to contain as few as possible sequences unrelated to the desired phenotype.

The resistance according to the invention is a resistance to white rust caused by *A. candida* race 9 Australian variant infection irrespective of the strain of Australian variant, contrary to the plant Booster, referred to in Petkowski *et al* and described in WO2010/135782 or the plant Tyson, referred to in Minchinton *et al.* In this respect, it is to be noted that, in fields, white rust infection is generally due to a plurality of different strains of *A. candida* race 9 Australian variant, stressing the importance of a resistance irrespective of the strain of Australian variant.

The seed or plant according to this aspect of the invention is preferably highly resistant to *A*. *candida* race 9 Australian variant, *inter alia* it remains free of symptoms till the end of the season when grown under natural infection conditions. In any case, a seed or plant of the invention, which is resistant to *A. candida* race 9 Australian variant has less than 25% of the leave surface with sporulation, and preferably less than 10% according to the pathology test described in example 1, and even preferably no sporulation on the leaf surface.

Plants or seeds of the invention, resistant to *A. candida* race 9 Australian variant, are also preferably resistant or tolerant to another pest, especially to pest of major agricultural importance.

Plants or seeds of the invention preferably also comprise other white rust resistance genes, preferably the monogenic dominant gene on chromosome 1 as disclosed in WO2010/135782, more preferably said plants or seeds comprise in their genome SEQ ID NO:1 and/or SEQ ID NO:2 as defined in WO2010/135782.

As detailed above, the invention is directed to *B. oleracea* broccoli plants, resistant to *A. candida* race 9 Australian variant infection, as well as to seeds giving rise to those plants.

*A B*. *oleracea* var. *italica* broccoli plant according to the invention may be a commercial plant or line or variety, preferably cultivated for its head. Such a commercial plant or line gives rise to head which are marketable, when grown in suitable conditions.

The plant according to invention may be an inbred or a dihaploid plant or a hybrid, and is a broccoli. It is preferably a cultivated *B. oleracea* broccoli.

The plant according to the invention may also be cytoplasmic male sterile, for example having the Ogura mitochondrial sterility.

Plants or seeds according to the invention are Broccoli plants (*B. oleracea* var. Italica). Alternatively, in a non-claimed embodiment, the description also concerns other *B. oleracea* plants, which are known as host for *A*. *candida* race 9 Australian variant, namely cauliflower (*B*. *oleracea* convar. Botrytis var. botrytis), Brussels sprouts (*B. oleracea* convar. Oleracea var. gemnifera), white cabbage, oxheart cabbage (*B. oleracea* convar. Capitata var. alba) and savoy cabbage (*B. oleracea* convar. Capitata var. sabauda). The introgressed sequences, conferring the resistance, can be transferred from a resistant broccoli, inter alia BROCCO-C4, to another *B. oleracea* plants, by a breeding program using the markers exemplified in the examples. A plant or seed of the invention is advantageously not a Romanesco plant or seed.

In this respect, it is to be noted that the inventors have, for the first time, identified sequences responsible for the resistance, have identified SNP markers associated with these sequences, and have proved that said sequences, initially present in a Romanesco plant can be transferred to another *B. oleracea* plants, without loss of the resistance phenotype, and without causing any other detrimental phenotype. Once the inventors have demonstrated that such a resistance gene exists, can be transferred from one genetic background to another one, while conserving its ability to confer resistance (penetration) without imparting negative features and once they have identified genetic markers linked to this resistance, it is then possible by a breeding program to transfer said sequences to another *B. oleracea* plants, and thus to cauliflower, Brussel sprouts, white cabbage and savoy cabbage.

A plant or seed according to the invention may be a progeny or offspring of a plant grown from the deposited seeds of *B. oleracea* var. Italica-BROCCO-C4, NCIMB 42433. Plants grown from the deposited seeds are indeed resistant to *A. candida* race 9 Australian variant, they thus bear in their genome the introgressed sequences conferring resistance. They can be used, in a non-claimed aspect, to transfer these sequences in another background by crossing and selfing and / or backcrossing, and selecting the plants of interest.

The invention is also directed to the deposited seeds *B. oleracea* var. Italica -BROCCO-C4 (NCIMB 42433) and to plants grown from one of these seeds. These seeds contain heterozygously the introgressed sequence conferring the phenotype of interest.

The presence of the introgressed sequences according to the invention can be revealed by the sequencing of the SNP identified by the inventors, and more specifically by allele C of SNP BO-0108746. The heterozygous status of the genome of a plant, seed or plant part with respect to the introgressed sequences of interest can be brought to light by the simultaneous presence of alleles C and A of SNP BO-0108746. The homozygous status of the genome of a plant, seed or plant part with respect to the introgressed sequences of interest can be brought to light by the presence of only allele C of SNP BO-0108746.

The invention also concerns any broccoli plant likely to be obtained from seed or plants of the invention as described above, and also plant parts of such a plant, and most preferably explant, scion, cutting, seed, root, rootstock, pollen, ovule, embryo, siliqua, protoplast, leaf, anther, stem, petiole, head and any other plants part, wherein said plant, explant, scion, cutting, seed, root, rootstock, pollen, ovule, embryo, siliqua, protoplast, leaf, anther, stem, petiole, head and/or plant part is obtainable from a seed or plant according to the first aspect of the invention, i.e. bearing the introgressed sequences of interest in their genome either homozygously or heterozygously. These plant parts, *inter alia* explant, scion, cutting, seed, root, rootstock, pollen, ovule, embryo, siliqua, protoplast, leaf, anther, stem, petiole or head, comprise in their genome the introgressed sequences conferring the resistance to white rust caused by *A. candida* race 9 Australian variant. The introgressed sequences referred to in this aspect of the invention are those defined above in the context of plants of the invention. The different features of the introgressed sequences defined in relation with the first aspect of the invention apply *mutatis mutandis* to this aspect of the invention. The introgressed sequences are thus preferably chosen from those present in the genome of a plant corresponding to the deposited material B. *oleracea* var. Italica -BROCCO-C4 (NCIMB accession number 42433) and conferring the resistance. They are advantageously characterized by the presence of allele T of SNP BN-0067786 (SEQ ID No:14) and/or allele C for SNP BO-0108746 (SEQ ID No:6), preferably allele C for SNP BO-0108746. The invention is also directed to cells of B. *oleracea* var. Italica cells, such that these cells comprise, in their genome, introgressed sequences conferring the phenotype of interest. The introgressed sequences are those already defined in the frame of the present invention, they are characterized by the same features and preferred embodiments already disclosed with respect to the plants and seeds according to the preceding embodiments of the invention. The presence of these introgressed sequences can be revealed by the techniques disclosed above and well known to the skilled reader. It can *inter alia* be determined whether the introgressed sequences are present homozygously or heterozygously in the genome of such a cell of the invention. They are advantageously characterized by the presence of allele T for SNP BN-0067786 (SEQ ID No:14) and/or allele C for SNP BO-0108746 (SEQ ID No:6), preferably allele C for SNP BO-0108746. A plant, explant, scion, cutting, seed, root, rootstock, pollen, ovule, embryo, siliqua, protoplast, leaf, anther, stem, petiole, head and/or plant part as defined above preferably comprises at least one cell of the invention, preferably the majority of the cells thereof are cells according to the invention, more preferably all cells of the plant part as defined are cells of the invention.

Cells according to the invention can be any type of *B. oleracea* var. Italica cell, *inter alia* a cell capable of regenerating a whole *B. oleracea* plant, bearing the introgressed sequences. Alternatively, the cell according to the invention can be a cell specifically not capable of regenerating a whole *B. oleracea* plant.

The cell according to the invention may be an isolated cell.

The present invention is also directed to a tissue culture of regenerable cells of the plant as defined above according to the present invention; preferably, the regenerable cells are derived from embryos, protoplasts, meristematic cells, callus, pollen, leaves, anthers, stems, petioles, roots, root tips, siliqua, seeds, flowers, cotyledons, and/or hypocotyls, and contain in their genome introgressed sequences on chromosome 4 conferring resistance to white rust caused by *A. candida* race 9 Australian variant.

The tissue culture will preferably be capable of regenerating plants having the physiological and morphological characteristics of the foregoing broccoli plant, and of regenerating plants having substantially the same genotype as the foregoing broccoli plant. The present invention also provides broccoli plants regenerated from the tissue cultures of the invention.

The invention also provides a protoplast of the plant defined above, or from the tissue culture defined above, said protoplast containing said introgressed sequences conferring resistance to *A. candida* race 9 Australian variant.

According to another non claimed aspect, the present invention is also directed to the use of a *B. oleracea* broccoli plant as detailed according to the first aspect of the invention, i.e. resistant, as a breeding partner in a breeding program for obtaining *B. oleracea* plants resistant to white rust caused by *A. candida* race 9 Australian variant. Indeed, such a plant according to the first aspect harbors in its genome introgressed sequences conferring the phenotype of interest, i.e. resistance. By crossing this plant with susceptible or less resistant plants, it is thus possible to transfer these sequences, conferring the desired phenotype, to the progeny as the phenotype is a monogenic trait. Preferably, a plant to be used as a breeding partner is a plant homozygous with respect to the introgressed sequence conferring the resistance, such that all hybrid plants resulting from the crossing of this plant with another *B. oleracea* will also bear the introgressed sequence conferring the resistance phenotype. Alternatively, a heterozygous plant according to the first aspect of the invention can also be used; in this case a selection step will advantageously be added, for example based on the presence of the SNP markers of the invention, in view of the segregation of the phenotype.

A plant according to the invention can thus be used as a breeding partner for introgressing sequences conferring the desired phenotype into a *B. oleracea* plant or germplasm.

The introgressed sequences of interest will advantageously be introduced into plant or varieties that contain other desirable genetic traits such as resistance to other diseases.

The introgressed sequences of interest will advantageously be introduced into plant or varieties that contain other white rust resistance genes such as the monogenic dominant gene on chromosome 1 as disclosed in WO2010135782; more preferably they comprise in their genome SEQ ID NO:1 and/or SEQ ID NO:2 as defined in WO2010/135782.

The description is also directed, in a non-claimed embodiment, to the same use with plants or seed of *B. oleracea* var. Italica -BROCCO-C4, deposited at the NCIMB under the accession number NCIMB 42433, or progeny thereof bearing the introgressed sequences of the invention conferring the resistance to white blister rust, especially progeny thereof having allele C of SNP BO-0108746. Said plants are also suitable as introgression partners in a breeding program aiming at conferring the desired phenotype to *B. oleracea* plant or germplasm, although it is heterozygous for the introgression sequences of interest, i.e. for obtaining *B. oleracea* plants resistant to white blister rust caused by *A. candida* race 9 Australian variant.

In such a breeding program, the selection of the progeny displaying the desired phenotype, or bearing sequences linked to the desired phenotype, can advantageously be carried out on the basis of the alleles of the SNP markers. The progeny is preferably selected on the presence of allele C of SNP BO-01 08746 on chromosome 4. The selection can alternatively be made on the basis of the simultaneous presence of allele T of SNP BN-0067786 and allele C of SNP BO-0108746.

Alternatively, the other SNPs of the invention, namely SNP BO-0108436, SNP BO-0108747, SNP BO-0108748, SNP BO-0108749, SNP BO-0108750, SNP BO-0108751, SNP BO-0108752, SNP BO-0108753 and SNP BN-0067793 can also be used as detailed above.

The selection of the progeny having the desired phenotype can also be made on conditions of *A. candida* race 9 Australian variant infection, as disclosed *inter alia* in example 1 (pathology test).

A plant according to the invention, or grown from a seed as deposited under accession number NCIMB 42433, is thus particularly valuable in a marker assisted selection for obtaining commercial broccoli lines and varieties resistant to white rust caused by *A. candida* race 9 Australian variant, as well as progeny thereof bearing the introgressed sequences of the invention conferring the resistance to white blister rust, especially progeny thereof having allele C of SNP BO-0108746.

The invention is also directed to the use of said plants in a program aiming at identifying, sequencing and / or cloning the genes conferring the desired phenotype, i.e. resistance to white blister rust caused by the oomycete *Albugo candida* race 9 Australian variant.

Any specific embodiment described for the preceding aspects of the invention is also applicable to this aspect of the invention, especially with regard to the features of the introgressed sequences conferring the resistance.

According to still another non-claimed aspect, the invention also concerns methods or processes for the production of *B. oleracea* plants, preferably *Brassica oleracea* var. *italica* broccoli plants, having the desired phenotype, especially commercial plants.

The present invention is indeed also directed to transferring the introgressed sequences conferring the resistance, from the broccoli plant to other *B. oleracea* varieties and species, especially to other broccoli species or to cauliflower (*B. oleracea* convar. Botrytis var. botrytis), Brussels sprouts (*B. oleracea* convar. Oleracea var. gemnifera), white cabbage, oxheart cabbage (*B. oleracea* convar. Capitata var. alba) and savoy cabbage (*B. oleracea* convar. Capitata var. sabauda); the invention is useful for producing new types and varieties of A. *candida* race 9 Australian variant resistant broccoli.

A non-claimed method or process for the production of a plant having these features, i. e. resistant to *A. candida* race 9 Australian variant, may comprise the following steps:
a) Crossing a plant according to the first aspect of the invention, and a susceptible or less resistant *B. oleracea* plant, in which the desired phenotype is to be imported or improved, preferably a broccoli plant,
b) Selecting a plant in the progeny thus obtained bearing the sequence conferring resistance,
c) Optionally self-pollinating one or several times the plant obtained at step b) and selecting a resistant plant in the progeny thus obtained;
wherein SNPs markers are preferably used in steps b) and c), for selecting plants bearing sequences conferring resistance to white rust caused by *A. candida* race 9 Australian variant. The SNP markers are preferably one or more of the 11 SNP markers of the invention, and preferably SNP BO-0108746. According to a preferred embodiment, the selection is at least partly carried out by detecting the alleles of SNP BN-0067786 and SNP BO-0108746. Alternatively, the selection can be made on the detection of the allele of at least 2 SNPs chosen amongst SNP BO-0108436, SNP BO-0108747, SNP BO-0108748, SNP BO-0108749, SNP BO-0108750, SNP BO-0108751, SNP BO-0108752, SNP BO-0108753 and SNP BN-0067793, preferably at least 3 SNPs, for example at least 4, 5 or 6, one of them being SNP BO-0108746. The selection can also be made on the detection of the alleles of all these 11 SNPs. The plant, which is selected at the selection step disclosed above, is preferably selected on the presence of allele C of SNP BO-0108746.

Preferably, the susceptible or less resistant B. *oleracea* plant of step a) is an elite line, used in order to introduce commercially desired traits or desired horticultural traits. It is preferably a broccoli plant.

A method or process for the production of a plant having these features may also comprise the following additional steps:
d) Backcrossing the resistant plant selected in step c) with a susceptible *B. oleracea* plant;
e) Selecting a plant in the progeny bearing the sequence conferring resistance to *A. candida* race 9 Australian variant,
f) Self-pollinating the plant obtained at step e) or crossing distinct plants obtained at step e), and
g) Selecting a plant resistant to *A. candida* race 9 Australian variant.

The plant selected at step b), c) or g) of the preceding method may be a commercial plant.

Steps d), e) and / f) may be repeated twice or three times or more, not necessarily with the same susceptible *B. oleracea* plant. Said susceptible *B. oleracea* plant is preferably a breeding line. This plant is preferably an elite line, used in order to introduce commercially desired traits or desired horticultural traits. It is preferably a broccoli plant.

The self-pollination/crossing and backcrossing steps may be carried out in any order and can be intercalated.

Moreover, these non-claimed methods are advantageously carried out by using SNP markers for one or more of the selection steps for selecting plants bearing the introgressed sequences linked to the resistance, or for selecting plants having the phenotype of interest.

A method for the production of resistant *B. oleracea* broccoli plants thus advantageously comprises the steps of:
a) Crossing a plant according to the first aspect of the invention and a susceptible B. *oleracea* broccoli plant,
b) Selecting one resistant plant in the progeny thus obtained, bearing the sequences conferring the resistance to white blister rust;
c) Optionally self-pollinating one or several times the resistant plant obtained at step b) and selecting a plant resistant to white blister rust in the progeny thus obtained;
d) Optionally backcrossing the resistant plant selected in step b) or c) with a susceptible B. *oleracea* broccoli plant and selecting a plant resistant to white blister rust,
wherein SNPs markers are used in steps b), c) and/or d) for selecting plants resistant to white rust, as disclosed herein.

The SNP markers are preferably one or more of the 11 SNP markers of the invention, and preferably SNP BO-0108746. According to a preferred embodiment, the selection is at least partly made on the basis of the allele of SNP BO-0108746 on chromosome 4. The selection is for example carried out by detecting the alleles carrying/characterized by SNP BN-0067786 and SNP BO-0108746.

The plant selected at the selection step disclosed above is preferably selected on the presence of allele C of SNP BO-0108746. In order to identify plants bearing homozygously the introgressed sequences responsible for the resistance, the presence of allele C of SNP BO-0108746 is detected in combination with the absence of allele A of SNP BO-0108746. Alternatively, the other SNPs of the invention can also advantageously be used, namely SNP BO-0108436, SNP BO-0108747, SNP BO-0108748, SNP BO-0108749, SNP BO-0108750, SNP BO-0108751, SNP BO-0108752, SNP BO-0108753, SNP BN-0067786 and SNP BN-0067793, in place or advantageously in addition to SNP BO-0108746.

The selection of the progeny having the desired phenotype can also be made on conditions of *A. candida* race 9 Australian variant infection, as disclosed *inter alia* in example 1.

The method used for allele detection can be based on any technique allowing the distinction between two different alleles of a SNP, on a specific chromosome locus.

Preferably, the plant according to the first aspect of the invention used in step a of the methods is a plant obtained by germinating a deposited seed BROCCO-C4 NCIMB accession number 42433 or progeny thereof bearing the sequences conferring the resistance to white blister rust, especially progeny thereof having allele C of SNP BO-0108746.

According to another non-claimed embodiment, the invention also provides a method for the production of *B. oleracea* plants, preferably broccoli, resistant to white rust caused by the oomycete *Albugo candida* race 9 Australian variant, comprising the steps of:
a1) Crossing a plant according to the first aspect of the invention or a plant grown from a deposited seed NCIMB 42433 and a susceptible *B. oleracea* plant, preferably broccoli, thus generating the F1 population,
a2) Advancing the F1 population to create F2 population,
b) Selecting one resistant plant in the progeny thus obtained;
c) Optionally self-pollinating one or several times the resistant plant obtained at step b) and selecting a plant resistant to white rust in the progeny thus obtained;
d) Optionally backcrossing the resistant plant selected in step b) or c) with a susceptible *B. oleracea* plant, preferably broccoli, and selecting a plant resistant to white rust,
wherein SNPs markers are used in steps b), c) and/or d) for selecting plants resistant to white rust, as disclosed herein.

The description also provides, in a non-claimed embodiment, a method for obtaining commercial broccoli plants, resistant to white blister rust caused by *A. candida* race 9 Australian variant, comprising the steps of:
a) Backcrossing a plant obtained by germinating a deposited seed BROCCO-C4 NCIMB accession number 42433 or a plant according the first aspect of the invention, with a *B. oleracea* broccoli plant susceptible to said white blister rust,
b) Selecting a plant resistant to white blister rust caused by the oomycete *Albugo candida* race 9 Australian variant.

According to a preferred embodiment of the methods, the selection of a resistant plant is carried out by detection of at least one of the following alleles: allele G of BO-0108436, allele C of BO-0108746, allele G of BO-0108747, allele A of BO-0108748, allele C of BO-0108749, allele A of BO-0108750, allele G of BO-0108751, allele C of BO-0108752, allele C of BO-0108753, allele T of BN-0067786 and allele G of BN-0067793, preferably by detecting allele C of BO-0108746. Preferably, the plant according to the first aspect of the invention used in step a1) or a) of these methods is a plant obtained by germinating a deposited seed BROCCO-C4 NCIMB accession number 42433, or progeny thereof bearing the sequences conferring the resistance to white blister rust, especially progeny thereof having allele C of SNP BO-0108746.

The present invention also concerns a *B. oleracea* var. Italica broccoli plant obtained or obtainable by one of the methods described above. Such a plant is indeed a *B. oleracea* var. *italica* plant having the desired phenotype according to the first aspect of the invention, i.e. resistant to white rust caused by *A. candida* race 9 Australian variant.

The invention is moreover directed to a method for detecting and/or selecting *B. oleracea* var. *italica* broccoli plants having introgressed sequences conferring resistance to white rust caused by *A. candida* race 9 Australian variant, on the basis of the allele detection. Such a method is based on the detection of the allele(s) of at least one of the 11 SNPs of the invention, especially based on the detection of the allele which is associated with the resistance, for at least one of the SNPs of the invention. In this respect, it is noted that, for the 11 SNPs of the invention, detection of the allele associated with the resistance is, on its own, indicative of the resistance phenotype, irrespective of whether the allele associated with the susceptibility is also detected, whereas the absence of detection of the allele associated with the resistance is indicative of susceptibility. The detection of the "susceptible" allele is not informative on its own. The method thus advantageously comprises the detection of whether at least one of the following alleles is present in the plant under test: allele G of BO-0108436, allele C of BO-0108746, allele G of BO-0108747, allele A of BO-0108748, allele C of BO-0108749, allele A of BO-0108750, allele G of BO-0108751, allele C of BO-0108752, allele C of BO-0108753, allele T of BN-0067786, and allele G of BN-0067793, the detection of at least one of these alleles allowing to conclude that the plant under test is resistant to white rust caused by *A. candida* race 9 Australian variant. Preferably, the method comprises the detection of the alleles of at least one SNP chosen amongst SNP BN-0067786 and SNP BO-0108746 on chromosome 4, preferably SNP BO-0108746, whereas detection of allele C of BO-0108746 is indicative of resistance, and/or allele T of SNP BN-0067786. The method can be carried out on *B. oleracea* plants resistant to white rust; the method thus can be used to confirm that such plants comprise in their genome the introgressed sequences according to this invention, and thus have been obtained according to the present invention.

Preferably, plants bearing the introgressed sequences are selected if allele C of SNP BO-0108746 is detected in a genetic material sample of the plant to be selected. The allele of interest can be present homozygously or heterozygously in the selected plant.

According to a particularly preferred embodiment, the selection is thus made on the simultaneous presence of allele T of SNP BN-0067786 and allele C of SNP BO-0108746. For selection of plants displaying homozygously the phenotype of resistance, the selection is preferably made on the simultaneous detection of allele T of SNP BN-0067786 and allele C of SNP BO-0108746 in combination with no detection of any other allele for these SNPs, and especially no detection of allele C of SNP BN-0067786 or allele A of SNP BO-0108746. Conversely, the detection of both alleles of a SNP is indicative of a heterozygous plant with respect to the resistance sequences.

Such a combination of alleles is to be found in plants grown up from to the deposited seeds.

The method of the invention allows selection of plants bearing the resistance sequences homozygously, by detecting plants only displaying the allele associated with the resistance for a given SNP of the invention, and also of plants bearing these sequences heterozygously, by detecting plants displaying both alleles associated with the resistance and with the susceptibility. The method also allows to make a distinction between these two types of resistant plants, those bearing the sequences heterozygously being characterized by the simultaneous presence of both the alleles associated with the resistance and with the susceptibility, for at least one of the SNPs of the invention.

The detection or selection may also comprise the identification of the alleles of at least one of SNP BO-0108436, SNP BO-0108747, SNP BO-0108748, SNP BO-0108749, SNP BO-0108750, SNP BO-0108751, SNP BO-0108752, SNP BO-0108753, SNP BN-0067786 and SNP BN-0067793, or at least 2, 3, or all 4 SNPs, or the identification of the alleles of at least one of SNP BO-0108436, SNP BO-0108747, SNP BO-0108748, SNP BO-0108749, SNP BO-0108750, SNP BO-0108751, preferably in addition to SNP BO-0108746.

The method of the invention is thus suitable for detecting and/or selecting *B. oleracea,* especially broccoli plants, having introgressed sequences conferring resistance to white blister rust caused by the oomycete *A. candida* race 9 Australian variant, e.g. as found in the genome of a plant of BROCCO-C4 (NCIMB accession number 42433), comprising detection of at least one of the following alleles: allele G of BO-0108436, allele C of BO-0108746, allele G of BO-0108747, allele A of BO-0108748, allele C of BO-0108749, allele A of BO-0108750, allele G of BO-0108751, allele C of BO-0108752, allele C of BO-0108753, allele T of BN-0067786 and allele G of BN-0067793, in a genetic material sample of the plant to be selected and/or detected. Detection of allele C of BO-0108746 is highly preferred.

The SNPs markers of the invention can also be used to map the resistance locus, such that the smallest possible introgression conferring the phenotype can be identified and/or selected.

In addition to introgression of the sequences conferring resistance, as detailed in the non-claimed methods of the description, these sequences of the invention can also be introduced into *B. oleracea* background by genetic engineering in order to obtain a commercial *B. oleracea* plant resistant to white rust caused by *A. candida* race 9 Australian variant. The identification and cloning of the introgressed sequences conferring the desired phenotype, *inter alia* from the deposit NCIMB 42433, can be carried out by the skilled person, on the basis of the sequence information given in the present application and deposited material. The description also concerns the genetic manipulations aiming at cloning the introgressed sequences conferring the phenotype, and/or integrating them into a *B. oleracea* plant, as well as the plants thus obtained, preferably broccoli plants.

According to a further aspect, the present invention is also directed to hybrid broccoli plants of *B. oleracea,* obtainable by crossing a resistant plant according to the first aspect of the invention, or a resistant plant obtainable by the methods disclosed above, with a plant of *B. oleracea,* for example a plant susceptible to white rust caused by *A. candida* race 9 Australian variant, or a plant with a different level of resistance to white rust caused by *A. candida* race 9 Australian variant. The resistant plant is preferably homozygous for the sequences conferring the resistance. If the resistant plant is heterozygous for the sequences conferring the resistance, only half of the progeny will be resistant; a selection step of the plants bearing the sequence conferring the resistance is thus preferably to be added.

A particularly preferred hybrid *B. oleracea* plant, is a plant which displays male sterility, or any other trait or phenotype of agronomical interest.

The invention also provides a method of protecting a field of *B. oleracea* plants, namely broccoli plants, against infection by *A. candida* race 9 Australian variant, said method comprising planting seeds according to the invention, or growing plants according to the invention, exhibiting the resistance phenotype.

### Examples:

### Example 1 : Identification of a resistant plant.

In a field trial held in Brittany, France, a romanesco *Brassica oleracea* plant showed resistance under high natural *Albugo candida* pressure.

The present inventors then decided to try this very same romanesco *B. oleracea* plant in a field trial in Australia so to check whether the resistance phenotype seen in Brittany could sustain the *Albugo candida* Australian variant pressure, although romanesco *Brassica* oleracea plants are not known to be the preferred host of *A. candida* race 9 Australian variant.

The results were positive and it was thought that the resistance determinism to both European and Australian variants was possibly driven by a unique genetic sequence.

Preliminary mapping work together with pathology tests did however show otherwise, as the mapping work on segregating populations identified two different localizations, on two different chromosomes. The genetic sequences conferring the resistance to the *Albugo candida* Australian variant of race 9 did therefore not confer resistance to the European variant. The initially tested romanesco plants probably possessed both sequences.

### Creation of a broccoli resistant plant:

The present inventors crossed the original romanesco *B. oleracea* plant with a broccoli inbred line to obtain a first generation hybrid, that was further backcrossed twice to the broccoli inbred line. The plants obtained thereof have been screened through the pathology test described hereunder, with an European *A. candida* race 9 strain and a symptomless plant was kept for the next steps backcross steps. Next generation plants were again tested in pathology test, this time with an Australian *A. candida* race 9 variant.

One of the resistant plants was used in a dihaploid program to produce a resistant inbred line homozygous for the resistance to the Australian *A. candida* race 9 variant. This plant has been further crossed to a susceptible broccoli plant and the resulting hybrid progeny plants BROCCO-C4, harbouring the resistance in a heterozygous way are resistant to the Australian *A. candida* race 9 variant. Seeds thereof were deposited at the NCIMB on 9^{th} July 2015 under accession number NCIMB 42433.

### Identification of the resistance:

In order to better identify the nature of the resistance to the Australian variant as well as to map the genetic location of this new resistance, a test was set up, made of 5 different F2 populations; indeed the original romanesco resistant plants were crossed to 5 different susceptible broccoli plants and the resulting F1 plants have been self-pollinated to produce F2 seeds.

### Pathology test :

An Australian variant *A. candida* strain has been collected in Templestowe, Australia and is kept in the freezer at -80°C. Every 2-3 months, it is periodically multiplied on susceptible varieties : the frozen cotyledons containing the pustules are washed with demineralized water to suspend the spores and the suspension is then vaporized on susceptible plants (cotyledon stage, 1 week after sowing) placed on growing chambers (16°C during the night, 15°C during the day, with 12 hours for each condition).

The cotyledon with the fresh, new pustules are collected into an Erlenmeyer flask together with 500 ml of distilled water and the solution adjusted at 2.10⁵ spores/ml in 2.5l of distilled water, thereby creating the inoculum.

The plant material is composed of 192 plants per genotype to be tested (i.e. 192 plants per F2 population), together with one susceptible and one resistant controls : seeds are sown and placed in greenhouses for about 3 weeks, up to the 1-2 leaves stage. Then, the leaves are gently rubbed to remove the leaf wax and the inoculum is sprayed on them. The reading is carried out 15 days after the spray, the plants being cultivated in growing chambers (16°C during the night, 15°C during the day, with 12 hours for each condition).

The reading is carried out with a 1 to 9 scale, where "1" corresponds to leaves with more than 75% of the surface with sporulation, "3" 51-75%, "5" is 26 to 40% of sporulation, "7" is 13 to 25%, "8" is 1 to 12% and "9" is no sporulation. The results are presented in table 2.

**Table 2:**

| Population | Total nb of plants | nb of plants scored 9 | nb of plants scored 8 | nb of plants scored 7 | nb of plants scored 5 | nb of plants scored 3 | nb of plants scored 1 |
|---|---|---|---|---|---|---|---|
| F2 population n°3 | 189 | 154 | 0 | 0 | 8 | 20 | 7 |
| F2 population n°2 | 191 | 149 | 0 | 9 | 7 | 22 | 4 |
| F2 population n°1 | 187 | 141 | 1 | 3 | 22 | 19 | 1 |
| F2 population n°4 | 183 | 135 | 0 | 2 | 13 | 27 | 6 |
| F2 population n°5 | 186 | 141 | 0 | 2 | 15 | 25 | 3 |
| Total over the 5 populations | 936 | 720 | 1 | 16 | 65 | 113 | 21 |
| susceptible parental line for population N°3 | 34 | 0 | 0 | 0 | 3 | 15 | 16 |
| susceptible parental line for population N°2 | 33 | 0 | 3 | 14 | 14 | 2 | 0 |
| susceptible parental line for population N°1 | 4 | 0 | 0 | 0 | 1 | 3 | 0 |
| susceptible parental line for population N°4 | 36 | 0 | 0 | 0 | 7 | 27 | 2 |
| susceptible parental line for population N°5 | 34 | 0 | 1 | 0 | 0 | 24 | 9 |
| Resistant plant used as control | 29 | 29 | 0 | 0 | 0 | 0 | 0 |

Some seeds did not germinate so that the exact number of plants per population was slightly lower than 192.

All results are in favour of a 3:1 segregation ratio demonstrating that the resistance to the *A*. *candida* Australian variant acts as a dominant one. The population n°3 shows a slight deviation at 5%, but not at 1% (significance assessed by a Chi-Square test).

A chi-square test was performed using was performed using R statistical software (http://www.r-project.org/) to confirm the hypothesis of one dominant resistance gene. The results are presented in table 3.

**Table 3:**

| | Number of plants scored 9 | Number of plants scored 1 to 8 | p-value ChiSquare test (segregation hypothesis 3:1) |
|---|---|---|---|
| F2 population n°3 | 154 | 35 | 0.04 |
| F2 population n°2 | 149 | 42 | 0.337 |
| F2 population n°1 | 141 | 46 | 0.899 |
| F2 population n°4 | 135 | 48 | 0.701 |
| F2 population n°5 | 141 | 45 | 0.799 |

### Example 2 : Genetic analysis :

Each F2 plant was sampled individually and DNA was isolated, using magnetic beads (NucleoMag^{®} 96 Plant), according to the protocol of the manufacturer of the beads, Macherey-Nagel.

For each of the 4 F2 populations that best followed the 3:1 segregation ratio, i.e. populations n°1, 2, 4 and 5, 22 F2 plants were genotyped with 384 SNP markers well spread over the B. *oleracea* genome, using Illumina Veracode Golden Gate technology. For each of the 4 populations, among the 22 plants, 11 of them were randomly taken among the plants scored 9, and 11 of them were taken among the plants scored 1. If, for a given population, less than 11 plants had been scored 1, all the plants scored 1 were genotyped and additional plants were taken among the plants scored 3 to make a total of 11 susceptible plants genotyped.

A QTL detection was performed across the 4 populations (22 x 4 = 88 individuals in total) using a Kruskal-Wallis test (R statistical software http://www.r-project.org/). Results showed that resistance locus was located on chromosome 4 (C4) between position 2 780 692 and 39 085 110, such physical position on the genome being based on the version V2.1 of the Brassica oleracea TO1000 genome published by the European Nucleotide Archive and EnsemblPLants - http://plants.ensembl.org/Brassica_oleracea/Info/Index.

The results are presented in table 4. The physical position (of the polymorphic nucleotide within the marker) is given with respect to the version V2.1 of the Brassica oleracea TO1000 genome, on chromosome 4. The sequences of the markers are detailed in table 11.

**Table 4:**

| Marker | Physical Position | K statistics | Degree of freedom | p-value | -log₁₀ **(p-value)** |
|---|---|---|---|---|---|
| BO-0003134 | 201 198 | 4.13 | 2 | 1.27E-01 | 2.06 |
| BN-0002724 | 2 780 692 | 27.01 | 2 | 1.36E-06 | 13.5 |
| BO-0019607 | 4 791 593 | 30.91 | 2 | 1.94E-07 | 15.45 |
| BN-0000514 | 10 159 531 | 63.09 | 2 | 2.00E-14 | 31.54 |
| BO-0003292 | 10 210 325 | 63.09 | 2 | 2.00E-14 | 31.54 |
| BN-0002556 | 12 815 918 | 65.05 | 2 | 7.50E-15 | 32.52 |
| BN-0003411 | 16 847 275 | 63.05 | 2 | 2.03E-14 | 31.53 |
| BN-0003048 | 22 390 855 | 44.14 | 2 | 2.60E-10 | 22.07 |
| BN-0001908 | 24430791 | 15.33 | 2 | 4.70E-04 | 7.66 |
| BN-0004578 | 24 889 844 | 25.47 | 2 | 2.95E-06 | 12.73 |
| BN-0003960 | 29 365 047 | 31.56 | 2 | 1.40E-07 | 15.78 |
| BN-0002472 | 32 235 933 | 9.78 | 2 | 7.53E-03 | 4.89 |
| BO-0000484 | 32 940 402 | 27.89 | 2 | 8.78E-07 | 13.95 |
| BN-0000937 | 33 185 730 | 7.62 | 2 | 2.21E-02 | 3.81 |
| BN-0009989 | 39 085 110 | 31.32 | 2 | 1.58E-07 | 15.66 |
| BO-0019453 | 42476727 | 5.95 | 2 | 5.11E-02 | 2.97 |
| BN-0001209 | 43 594 509 | 5.95 | 2 | 5.11E-02 | 2.97 |
| BN-0000561 | 47 686 738 | 9.35 | 2 | 9.33E-03 | 4.67 |
| BO-0025709 | 47 922 677 | 6.55 | 2 | 3.78E-02 | 3.27 |
| BN-0004240 | 50321 242 | 3.54 | 2 | 1.71E-01 | 1.77 |
| BN-0001712 | 51 192 932 | 2.74 | 2 | 2.55E-01 | 1.37 |
| BN-0004051 | 52 006 644 | 1.55 | 2 | 4.60E-01 | 0.78 |

### Validation of the markers :

### The Genotyping was done using KASP^{™} technology.

The most tightly linked markers identified were tested on all the plants of four populations. Additional markers, located in the same genome region but initially not in the set of 384 SNP used for discovery were also genotyped. F2 population n°1 was not tested in this first phase. A QTL detection was performed for each populations separately using a Kruskal-Wallis test (R statistical software http://www.r-project.org/). Table 5 shows the -log10 (p-value) value for each marker in one or more populations. NT means 'not tested' in table 5. Results showed that marker BN-0067786 located at position C4: 8 079 868 was the marker linked the more closely with the resistance, in view of the results obtained for F2 population n°2.

**Table 5:**

| | | -log10 (p-value) | | | | |
|---|---|---|---|---|---|---|
| Marker | Physical Position | F2 pop. n°2 | F2 pop. n°3 | F2 pop. n°4 | F2 pop. n°5 | F2 pop. n°1 |
| BN-0067786 | 8 079 868 | 50.56 | NT | NT | NT | NT |
| BN-0067793 | 8 081 104 | 49.53 | NT | NT | NT | NT |
| BN-0003120 | 8 712 484 | 48.06 | 80.80 | 52.54 | 60.47 | NT |
| BN-0050542 | 9 777 719 | 36.93 | 71.87 | NT | NT | NT |
| BN-0000514 | 10 159 531 | 47.63 | NT | 48.35 | 57.38 | NT |
| BO-0003292 | 10 210 325 | 0.68 | 0.69 | 1.15 | 0.84 | NT |
| BN-0002556 | 12 815 918 | 42.76 | NT | 46.57 | 53.68 | NT |
| BN-0051988 | 13 237 267 | 29.96 | NT | 36.54 | 45.07 | NT |
| BN-0003411 | 16 847 275 | 42.74 | NT | 44.13 | 50.88 | NT |

A second Genotyping experiment was done using KASP^{™} technology.

24 SNP located between positions C4: 6 069 705 and C4: 8 712 484 were screened for polymorphism among the two parental lines of F2 population n°1. 5 of them showed polymorphism and were further genotyped on the whole F2 population n°1. F2 population n°1 was thus tested in this 2^{nd} phase. One marker did not show the expected segregation and has thus been discarded from the analysis. Association test was performed using a Kruskal-Wallis test (R statistical software http://www.r-project.org/). The results are presented in table 6.

**Table 6:**

| Marker | Physical Position | K statistics | Degree of freedom | p-value | -log10 (p-value) On F2 pop n°1 |
|---|---|---|---|---|---|
| BN-0000253 | 6 069 705 | 123.22 | 2 | 1.75E-27 | 61.61 |
| BO-0103559 | 8 197 030 | 162.75 | 2 | 4.55E-36 | 81.38 |
| BO-0103567 | 8 195 495 | 162.75 | 2 | 4.55E-36 | 81.38 |
| BN-0003120 | 8 712 484 | 158.10 | 2 | 4.77 E-35 | 79.03 |

Phase 1 of the markers validation allowed refining the right boundary position of the resistance (i.e, downstream boundary of the interval according to coordinates along the chromosome), located on BN-0067793 at position C4: 8 081 104.

Phase 2 of the markers validation allowed refining the left boundary position of the resistance (i.e, upstream boundary of the interval according to coordinates along the chromosome), located on marker BN-0000253 at position C4:6 069 705.

It is noted that whereas phase 1 has been carried out on different populations, phase 2 has been carried out on a single population, namely F2 population n°1.

In order to determine the smallest introgression fragment, imparting the resistance, it must first be determined the highest -log p value, in a given population, and then to set the boundary of the introgression fragment to the nearest SNP showing a decrease in this value, preferably confirmed by results on another population.

The interval containing the resistance is thus spanning a 2 011 399 bp region between positions 6 069 705 and 8 081 104 on chromosome C4.

### Fine mapping :

Genotyping was done using KASP^{™} technology.

To further fine map the resistance locus, 11 SNPs located between positions C4: 7 366 478 and C4: 7 479 283 were screened for polymorphism among the parental lines of the 5 F2 populations. Only one of them (marker BO-0108436 located at position C4: 7 427 499) was polymorphic (in population n°3) and was further tested in the whole population n°3. Association test was then performed for marker BO-0108436 on population n°3 using a Kruskal-Wallis test (R statistical software http://www.r-project.org/). The results are presented in table 7.

**Table 7:**

| Marker | Physical Position | K statistics | Degree of freedom | p-value | -log10 (p-value) On F2 pop. N°3 |
|---|---|---|---|---|---|
| BO-0108436 | 7 427 499 | 121.31 | 2 | 4.55E-27 | 60.65 |

Thanks to the study of populations, and the overlap of confidence intervals, the resistance interval boundaries could be set up to: left boundary: C4: 7 427 499, corresponding to marker BO-0108436 and right boundary: C4: 8 081 104, corresponding to marker BN-0067793. The interval containing the resistance is thus spanning a 653 605 bp region between positions 7 427 499 and 8 081 104 on chromosome C4.

### Example 3 : Identification of further markers linked to the resistance to Albugo candida on chromosome C4.

The interval containing the resistance is spanning a 653 605 bp region between positions 7 427 499 and 8 081 104 on chromosome C4. Looking at the reference genome sequence mentioned herebefore, 73 genes were identified in this region. Polymorphisms within these genes were studied more closely, after amplification by PCR and sequencing the amplified region. The most promising amplified region was generated by using the following primers:
Forward Primer CGCTCAAACGGTTAACATGA (SEQ ID No:41)
Reverse Primer CCATCGCCACTTGACGTTAT (SEQ ID No:42)

PCR mix was prepared as follow:

| | |
|---|---|
| MilliQ Water | 4µL |
| 10X Buffer | 1.5 µL |
| dNTP 2,5mM | 1.5 µL |
| MgCl2 25 mM | 1.5 µL |
| Forward primer 10µM | 0.7 µL |
| Reverse primer 10µM | 0.7 µL |
| Taq Sigma Jump | 0.1 µL |
| DNA template | 5 µL |

PCR amplification was performed on a Applied Biosystems 2720 Thermal Cycler using the following program:
Step 1 : 3 min at 94°C; Step 2: 30 sec at 94°C; Step 3: 30 sec at 60°C; Step 4 :2 min at 72°C;
Step 5 : 7 min at 72°C
repeat steps 2 to 5 for 40 times
Step 6 Hold at 15°C

Amplification was performed on parental lines of the 5 populations (including the resistance source) and one recombinant plant of population n°1. This plant was scored 4 in the pathology test, has a susceptible parent profile upstream the resistance region and a heterozygous profile downstream the resistance region. As it is phenotypically susceptible, the plant is expected to carry the susceptible allele at the resistance gene location.

Sequencing of the amplicons was performed on both strands by GATC Biotech using Sanger sequencing technology. Sequences analysis was performed using BioEdit software before merging both strands sequences. No polymorphism could be identified among the 4 susceptible parents and the susceptible recombinant plant. Several nucleotide polymorphisms between the consensus susceptible sequence and the resistant source sequence were identified in the amplified fragment; out of them 8 SNPs were selected as particularly robust at distinguishing the resistant parent sequence from the susceptible parents consensus sequence and the reference sequence. The alleles of these markers are reported in table 8.

**Table 8:**

| Marker | Susceptible allele | Resistant allele |
|---|---|---|
| BO-0108746 | A | C |
| BO-0108747 | T | G |
| BO-0108748 | G | A |
| BO-0108749 | A | C |
| BO-0108750 | G | A |
| BO-0108751 | T | G |
| BO-0108752 | T | C |
| BO-0108753 | T | C |

### Markers validation in the mapping populations

Genotyping was done using KASP^{™} technology. Some of the markers were genotyped in the F2 segregating populations n°1 and 3.

Association test was performed on each population separately using a Kruskal-Wallis test (R statistical software http://www.r-project.org/). The results are presented in table 9.

**Table 9:**

| | | -log10 (p-value) | |
|---|---|---|---|
| | | F2 population n°1 | F2 population n°3 |
| Marker | Physical Position | | |
| BN-0000253 | 6 069 705 | 61.61 | |
| BO-0108436 | 7 427 499 | | 60.65 |
| BO-0108746 | 7 463 142 | 85.65 | 92.46 |
| BO-0108747 | 7 463 198 | | |
| BO-0108748 | 7 463 204 | 8.42 | |
| BO-0108751 | 7 463 248 | 85.05 | 90.57 |
| BO-0108752 | 7 463 339 | 6.24 | |
| BO-0108753 | 7 463 352 | 7.66 | |
| BO-0103567 | 8 195 495 | 81.38 | |
| BO-0103559 | 8 197 030 | 81.38 | |
| BN-0003120 | 8 712 484 | 79.03 | 80.80 |

The interval containing the resistance is thus with a very high probability in a region between positions 6 069 705 and 7 463 248 on chromosome C4.

### Example 4: Assessment of molecular makers in the breeding germplasm.

Genotyping was done using KASP^{™} technology. 488 different genotypes were sampled and DNA was isolated, using magnetic beads (NucleoMag^{®} 96 Plant), according to the protocol of the manufacturer of the beads, Macherey-Nagel. The 488 genotypes are constituted by:
- The initial Romanesco source of resistance,
- The deposited hybrid BROCCO-C4,
- 2 resistant lines, rendered resistant by introgression of the fragment of the invention,
- 51 commercial hybrids of broccoli
- 433 lines of broccoli.

Association test was performed for the marker BO-0108746 (SEQ ID N°6) using a Kruskal-Wallis test (R statistical software http://www.r-project.org/). A -log10 (p-value) of 243.5 (K statistic = 487) was found. A pearson correlation test was also performed (R statistical software http://www.r-project.org/). A correlation coefficient of 1.0 was observed at 95% confidence interval (t statistic = 853277176, p-value = 0.0).

These tests have shown a correlation of 100% between the genotype and the phenotype, i.e. all the resistant plants (Initial Romanesco, BROCCO-C4 and the 2 resistant lines) exhibit the presence of allele C of BO-0108746, whereas all the other plants, susceptible to *A. candida* race 9 Australian variant do not exhibit the presence of this allele, but only allele T of BO-0108746.

Marker BO-0108746 was confirmed as the most predictive SNP for identifying and selecting broccoli plants resistant to *A. candida* race 9 Australian variant.

### Example 5: genotyping of BROCCO-C4.

As detailed in example 1, a resistant plant obtained from an initial cross between a Romanesco *B. oleracea* plant and an inbred broccoli line, was used in a dihaploid program to produce a resistant inbred line homozygous for the resistance to the Australian *A. candida* race 9 variant. This plant has been further crossed to a susceptible broccoli plant and the resulting hybrid progeny plants BROCCO-C4, harbouring the resistance in a heterozygous way, are resistant to the Australian *A. candida* race 9 variant. Seeds of BROCO-C4 plants, giving rise to hybrid plant bearing the introgressed sequences linked to the resistance were filed at the NCIMB on 9^{th} July 2015, under accession number NCIMB 42433.

Plants of BROCCO-C4 were genotyped for the markers identified in examples 2-4. It was confirmed that BROCCO-C4 plants/seeds exhibit all the alleles of the SNPs shown above as linked to the resistance, as detailed in table 10.

**Table 10:**

| Marker | Resistance allele as defined in examples 2-4 | Presence of the resistance allele in BROCCO-C4 |
|---|---|---|
| BO-0108436 | G | Yes |
| BO-0108746 | C | Yes |
| BO-0108747 | G | Yes |
| BO-0108748 | A | Yes |
| BO-0108749 | C | Yes |
| BO-0108750 | A | Yes |
| BO-0108751 | G | Yes |
| BO-0108752 | C | Yes |
| BO-0108753 | C | Yes |
| BN-0067786 | T | Yes |
| BN-0067793 | G | Yes |

These results thus confirm that BROCCO-C4 plants are plants bearing the introgression fragment conferring the resistance to white rust caused by the *A. candida* race 9, Australian variant.

**Table 11: Marker sequences of all the SNP used in the examples, all on chromosome 4. The allele associated to the susceptibility (S) phenotype is indicated first within brackets, followed by the allele associated with the resistance (R) phenotype. It must be borne in mind that, outside the introgression fragment of the invention, delimited by SNP BO-0108436 and BN-0067793, preferably by SNP BO-0108436 and SNP BO-0108751, the association with susceptible/resistance phenotype is very low or absent.**

| Marker | Sequence Position | Sequence (S/R) |
|---|---|---|
| BO-0003134 | 201 198 | ATTTGATTGTGTGCCTTTCCCAGTTTATTGAATACAACATTGTT TCATGAAGCAGGTTGCAGGATTGCTTGGTG[T/C]GAGTTCTGA TCTTTCAGCTTACTGTCCTGGTCCAAGTCCTTCTTCAGCTGAT ACTGAAATCAAACCTGATCTGCA (SEQ ID No :1) |
| BN-0002724 | 2 780 692 | AGCTCTTGTCTCTGAAAGAACGTCAAACCTTCAAAAAATGGCA TTGAAGTTTAATCCTTTGGTATCTCAGCCAT[A/G]CAAACTCGC TTCCTCGGCTCGTCCGCCAATCTCTACTTTCAGATCTCCCAA GTTCCTCTGCCTCGCTTCTTCTTC (SEQ ID No :2) |
| BO-0019607 | 4791 593 | TTCAATATAAGCTGCAGAAAACAAGAGGTGGTAGGTAATAGA AAAAAAACCTGCTCAATGATCTCATGTCTCTG[C/A]AGGTTTCT TGAAAACCCTCCTTTCCCAGTAGCACAAAACGAGCAACGCAG TGGACATCCAACCTAAATCAGAGAG (SEQ ID No :3) |
| BN-0000253 | 6 069 705 | TACGAGTTTGGATTTTAACTTTTTATAAAAAAGTATAATTAAAA AATGAGCTTGAAGGACGCCAGCAAGGAATG[C/T]GGGTGATG AATGATGATCGTATCTTCCGTAAGATACGGAACACATAACGC CATCTATCTTCAGACAAAGACATTC (SEQ ID No :4) |
| BO-0108436 | 7 427 499 | CCCATGAAGTTGAGGTATCCTTCTCCACCTAGTCCCATCGTA ATGAGCCGTATCATTTTGTGTAAGGCGATCCC[A/G]CGGTCTA CACGTGGTGTGGCCTGTTTGTCCACCGCCATGAAATCATACA TGTCCTACAAAACAGTAAGATGTTAG (SEQ ID No :5) |
| BO-0108746 | 7 463 142 | TTAATGTTGATGAAGAACTAGAAGCCATGGGTAAGAAAATGG TCACATATTGTGGAGGACTCCCATTAGCCGTTA[A/C]AGTGCT GGGAGGCTTGTTAGCTAATAAAACCATGGTTGAAGAGTGGAA AAGAATATATGACGATATTCAAACTCA (SEQ ID No :6) |
| BO-0108747 | 7463 198 | GGACTCCCATTAGCCGTTAAAGTGCTGGGAGGCTTGTTAGCT AATAAAACCATGGTTGAAGAGTGGAAAAGAATA[T/G]ATGACG ATATTCAAACTCAGATAGTTCGCATAGATGAAAACAGTCAAGA TTCGGTTTACCGAGTATTGTCTCTAA (SEQ ID No :7) |
| BO-0108748 | 7 463 204 | CCATTAGCCGTTAAAGTGCTGGGAGGCTTGTTAGCTAATAAA ACCATGGTTGAAGAGTGGAAAAGAATATATGAC[G/A]ATATTC AAACTCAGATAGTTCGCATAGATGAAAACAGTCAAGATTCGG TTTACCGAGTATTGTCTCTAAGCTATG (SEQ ID No :8) |
| BO-0108749 | 7 463 236 | AGCTAATAAAACCATGGTTGAAGAGTGGAAAAGAATATATGA CGATATTCAAACTCAGATAGTTCGCATAGATGA[A/C]AACAGT CAAGATTCGGTTTACCGAGTATTGTCTCTAAGCTATGAAGATT TGCCAATGCAGTTAAAGCATTGCTTC (SEQ ID No :9) |
| BO-0108750 | 7 463 246 | ACCATGGTTGAAGAGTGGAAAAGAATATATGACGATATTCAAA CTCAGATAGTTCGCATAGATGAAAACAGTCAA[G/A]ATTCGGT TTACCGAGTATTGTCTCTAAGCTATGAAGATTTGCCAATGCAG TTAAAGCATTGCTTCCTCTACCTAG (SEQ ID No :10) |
| BO-0108751 | 7 463 248 | CATGGTTGAAGAGTGGAAAAGAATATATGACGATATTCAAACT CAGATAGTTCGCATAGATGAAAACAGTCAAGA[T/G]TCGGTTT ACCGAGTATTGTCTCTAAGCTATGAAGATTTGCCAATGCAGTT AAAGCATTGCTTCCTCTACCTAGCT (SEQ ID No :11) |
| BO-0108752 | 7 463 339 | TTGTCTCTAAGCTATGAAGATTTGCCAATGCAGTTAAAGCATT GCTTCCTCTACCTAGCTCATTTTCCCGAAGAT[T/C]ACAAGATA CAAGTGGAGAGATTGTATTATCTCTGGGAAGCAGAAGGAATA ATAACGTCAAGTGGCGATGGAGAAA (SEQ ID No :12) |
| BO-0108753 | 7 463 352 | ATGAAGATTTGCCAATGCAGTTAAAGCATTGCTTCCTCTACCT AGCTCATTTTCCCGAAGATTACAAGATACAAG[T/C]GGAGAGA TTGTATTATCTCTGGGAAGCAGAAGGAATAATAACGTCAAGT GGCGATGGAGAAACCACTAGGAAAAT (SEQ ID No :13) |
| BN-0067786 | 8 079 868 | AACTTGTCTTTGATTTATTTATGTTCGCTTTTTCTTTTGATACTA CAAAAGCTAATCATTTCCAAATCTCGAAA[C/T]GAAAGTCTAGC ATATGTACAACTCTTATGCATCTTTGGCAAAAAAAATGATTCT CGGAAAATTACATGTTCGTAT (SEQ ID No :14) |
| BN-0067793 | 8 081 104 | CGATACAGTTGTATGTACAGGCTAAAAAAATGTTTTTTTAAAG AAAAACATAAAACTATATAACTTGTTCCATA[A/G]TGAATACAT ACATAGTCACGTAGATCATTCGGATTTGGCAACTCTTCTTATC ATGCGCATAATCAAACCTCCAAA (SEQ ID No :15) |
| BO-0103567 | 8 195 495 | TAATTGATCAGAATCTTCACATCTTTTAAGAGAAACCAAAAAAT GGTCTACACACCATCCTCTTTTGCTTACTA[T/G]CATGCATGAT AAGTAGAAACAAAAGAAAAGAAGAAGCATCTCTATGTTTACAA AGTGGCAGCAACCAAATCAGGA (SEQ ID No :16) |
| BO-0103559 | 8 197 030 | ATGGAGGATACACCTTCCGTGTCCGGAGATAAGGTGAGAGC TTTACGCGCGTGATTCAGAGCTGAGATCAAGTC[G/A]CCGCT GTTGAATTTCGATTCCGCTAGGGTGTATTGCCTCGTTACCAG TGTCTCGGTCTGATCCTGAAGCTGCCAT (SEQ ID No :17) |
| BN-0003120 | 8 712 484 | TTCGTGAGCTGATTCTTGGTGGGGTATGTTGATTTGCCAGAT AACAATGAAAGTCATAAGAAGCAAGAACGTCC[A/C]GATTCCC AAACTCCACCACCGGTTCCTTCAGGACCATGTTCTGAATCTAT AGATACAAGCTCCGTCTACTCAGAA (SEQ ID No :18) |
| BN-0050542 | 9 777 719 | TTCTTTTTATCCGCCTTCTCAATTTGCCGATTTAGGGTTTCTCT TTTGGTTGATTTGTCGGCGTCACTGTTGTT[T/G]TTTTTATTCC GTACAGTTTCTGGAGAAGTTCCCGAGGTGCCTGTGGTTTCAA CGAAGTCAGGTTTGCTCTTCGAG (SEQ ID No :19) |
| BN-0000514 | 10 159531 | AATAACTGTAACATTTAAGACTCTTCTTGCTATAATAACTTATG TTTTCTGTTCCAGCTTGTTGACCCAAAGAC[A/G]GGAGATGAG AAACCCATTACAGTCTCTGTTGATGATGGGATCCGACCAAGC ACAACCCTTGCTACTCTTGGGAAG (SEQ ID No :20) |
| BO-0003292 | 10 210 325 | ACGAATCTCGTGATGGTCATCAAATCCGTACGGTAAGCATCT GCTTCGTGATCCATCTTTGTTTCTCGCTCTCC[T/C]CGTTATGA AGCGGATACCTTTTTAATCAGATGTTTAGAAGATATTATATATA ACCTGCATTATTTTTCTTTTTAA (SEQ ID No :21) |
| BN-0002556 | 12 815 918 | AACCTCTTGGGATGGATTCTTTTGGGAGTGTTTGGTTTGATCT GGACTTTCTACTTTACCTACACTTCTTCTCT[G/C]GACGAGGAT GAAGAATCTGGTCTTTCACTCTAAACATGAACTGTATATGTTC TTCATCAGTGTCATCTCTCTCCT (SEQ ID No :22) |
| BN-0051988 | 13 237 267 | AAAATAGAAAAAATTGAATAGTTGGTATTCAAACATTGATTCTA AGCTTGCCAAATTGATCCGGATCACTAGTT[T/C]TACTGGTTTT AGTCGGTAAAACCGGATATTTTCTTTAAATCAATTTTTAAACCA AACTGAACTCGGCTTATTCAA (SEQ ID No :23) |
| BN-0003411 | 16 847 275 | TCCGTTATGATACTTCAATCGCCTTTATTGGCATCGGAACAAA GAAACTCTCTGCCGACGAGGAGCAAACACGC[G/A]GAGAGGA GAAAAGTAGGAGAGGTAGCTGGAGGAGCGGCGGCCGAGTG TGCGGCGGTCTGGTGTTGCTGTCCTTGC (SEQ ID No :24) |
| BN-0003048 | 22 390 855 | ATTTCGAGCAACTCGCGAAGAAGCTGGAAGAGGCTTCTCCAC TTGAAATCATGGACAAAGCTCTCCAGAAATTC[A/G]GAAGCAA CATCGCAATTGCTTTCAGGTAATCAATCGATTCAATTAAAGAT CCAAAAGAATAGAAAGAACTTGAAT (SEQ ID No :25) |
| BN-0001908 | 24 430 791 | TATAGTTCCGTCTTAAGTTTGTTTACATGTACAATATGCTTTTA CGATTTCAGTCCTTGGCTTGAGGCACCCTA[T/C]AAAGATGCC GCAGAGAAAACACCGGGCGGAAAGTTGACAATCAATGGGTTT CTGTCTGAGGTTTGTATGCTTGCT (SEQ ID No :26) |
| BN-0004578 | 24 889 844 | ATTGTTTCTCTTCGTAGCTGCCACCGCAACGGCAGCCGCTAC ACCAACCAAACGCGCAAACTACCTTTTCACAC[C/G]GCACGCA AAAGCAGTCGCGGGAATATGCACTGTAATCCAAACAAACACA AGTCTATGCTGCAAAACCCTTAAACA (SEQ ID No :27) |
| BN-0003960 | 29 365 047 | TGCAAATGGCAGGCACACTATATCAGCGGCACAAGCAGCCTT TCTTGATGATCACTTGGGATCTATATCTCCAG[G/C]GAAGCTT GCGGATTTTGTTGTATTGTCAACAAATTCATGGGACGAGTTCT CAAAAGACGGTTCTGCTTCGGTTTT (SEQ ID No :28) |
| BN-0002472 | 32 235 933 | CTAGATTTATGTAATTTGAAGGGTTCAAATGTGACTGGTGGG GTTGGCCCTTTTGGTCTGATCACCTTGGCTAC[A/G]TCTGACC TACAAGAATACACTCCCGTCTTCTTCAGAGTCTTCAAAGATGG CACAACCAACAAGCCTAAGGTCCTC (SEQ ID No :29) |
| BO-0000484 | 32 940 402 | GAGTAAGTTAAAATCTAAAGACAAGGGCAAGTCTTCAAAAAA GAAGGAAGCAACGAGTAATGCAAAAGAAGGAC[C/T]GAGGAC ACCTGGAGGCGAAGAAGCGCTTTCAAATGTAACCAAGGAGAA GGCTGCTGCTGCCAAACTATATATTGA (SEQ ID No :30) |
| BN-0000937 | 33 185 730 | GCTGTCCCAGATATCTGGAGATCTTCTGCAGAAAAGTATGGG GATAAAATTGCACTTGTAGATCCTTACCATGA[C/T]CCACCTTT GAAATTGACATACAACCAGGTATATGTCTATTGTCGCTGTAGA CTTGGTGAGCGAGCAACGGAGTTC (SEQ ID No :31) |
| BN-0009989 | 39 085 110 | CGAAACCTCCAGCAGATTCCAGAAAGGTTGAAACTTCCTCAA AGCCCGAGACCAAGAAGAAGCGATCTAGTGAG[G/A]CAGATG AAGGCGAAGAAGCAAAGAGGGTCAAGAGGGTTTCAGGGGAA GACGACCACAAAAAGAGTAAGGGAGAGG (SEQ ID No :32) |
| BO-0019453 | 42476727 | ATTTCAATTCGTGTTATTGGAATCATACCTTTTTTTTCTTTCCA GGTGATGTTATTTGCCGGTTCGGAAACAAC[G/T]TCAGTGACA TTAGAGTGGGCTATGTCTAACCTTCTAAATCATCCAGATGTTC TAGCGAAAGTAAAGACGGAACTA (SEQ ID No :33) |
| BN-0001209 | 43 594 509 | GATTTTCAGTTTACACATCCCATCAATCACAATTATATTTATTA AAAAAAAAAGTGCTAACTTACAATCTTATC[A/G]AAGAGTTCTC CTCCTGTAACATACTCCAAGATGATAAATATTTTTGTCTTGCTT GCCATCACCTGCAAAATCACA (SEQ ID No :34) |
| BN-0000561 | 47686738 | AGTTTTAGATACAAAGAAGTGTCTCTCTGCAATGAAAGAACCG ATTTAAGAAAAGTACCAGCGGTTGTGGTGCC[G/A]TCCTTTTT AAACACAGGCTTCAGCTTCCCAAGAGTAGCAAGGGTTGTGCT TGGTCGGATCCCATCATCAACAGAA (SEQ ID No :35) |
| BO-0025709 | 47 922 677 | CTCACTTGTTATTTTCCACATCTTTTGTTCACTGAGATTAGTCC CTTTTGGCAGAATTGGTATTGTGCATCAAA[A/G]ACACAAGCA GAAAGTGAAGCTTTCGAATTTGCAAAACGAACCGGGATTAGT CTCGTCAGCATTTGCCCTACCATG (SEQ ID No :36) |
| BO-0103565 | 48 421 318 | TAAAACCCAAAGCCATATACCACTTCAACATTATATTATTTTTT ACGTTATTGGATTTAAAAGTCAACGACCTC[A/C]AAGTTCCATC AACCAATCTTTGTCGGAATACTCTGTTCATGCCTGAAAAAGTT CTCAGGATCAAACTCACCTTTA (SEQ ID No :37) |
| BN-0004240 | 50 321 242 | CAATCAAGATAATCCTCTGCATCTTCATAATCAGTGAGGACAG ACACCGTGCATTGCAGAAACGGCAGTTCTTT[C/G]GGTTGGAT TGGTGGGAACCTACGATCCCTCAGGGCACTGTGATATTGACA TAAATCAAAAAGGTACATTTATAAC (SEQ ID No :38) |
| BN-0001712 | 51 192 932 | GGCGGCCTCCCTTCAACCCGCATGCTTGGACCTCCACTTCG CCGGGAAACATCCTCCGCCTCTCAAACACACCA[A/G]CGCCA CCTTCCTCCGCTGCGTCTCCTCTCCGAATTTCCCCGAGCCTG ACTCGCCTGACGCCAATCTCACCAAAGA (SEQ ID No :39) |
| BN-0004051 | 52 006 644 | AGCGTTGGAAGAGCATCTTTCTGATACTTGTTTCCAAGCTCA GAGATCTTTCCCAGCCACAAGTTGTTCCAAGC[A/G]AATATAA CAGCGATCACAGATGGACCGAGGATCACTTGGTTAAGCAGAA CCTAACAAAGGGATCGATAAGATACA (SEQ ID No :40) |

### Example 6: Comparison of the resistance level of different plants.

Different plants, corresponding to 6 different genotypes, were grown and inoculated as described in example 1. The tested plants are as follows:
- A: Tyson, the variety referred to in Minchinton et al;
- B: F2 of Tyson;
- C: The Romanesco introgression partner used in the present invention;
- D: A Broccoli line according to WO2010135782 having a resistance gene on chromosome 1,
- E: Broccoli Hybrid CMS deriving from BROCCO-C4;
- F : Broccoli line deriving from BROCCO-C4.

The resistance assay was repeated 4 times, with 6 plants for each genotype A-F and in each repetition. The results are reported in table 12 below.

As can be deduced from these results, only the plants bearing the introgression fragment of BROCCO C4 on chromosome 4 (genotypes C, E and F) display resistance to *Albugo candida* race 9 Australian variant, namely high resistance.

In order to confirm that the plant Tyson, referred to in Minchinton et al, does not possess the introgression fragment of the present invention, the inventors have moreover determined the allele of SNP BO-0108746 present in Tyson. The plant Tyson is homozygous for the allele A of BO-0108746, contrary to the plants of the invention, bearing allele C of this SNP.

### Example 7: Field trials.

Different hybrids, derived from the deposited seeds were obtained and tested in field trials, with other commercial hybrids. The field trials were carried out in autumn of two consecutive years, in Australia. Tables 13 (autumn of year N) and 14 (autumn of year N+1) report, for every plants, the total number of plants grown, the number of infected plants with white rust and the percentage of plants infected. Of note, a particularly stringent test of resistance was adopted in these field trials, a plant was indeed considered as being "infected by white rust" when it has a score of strictly less than 9 in the pathology test of example 1 and only the plants having no spores at all, i.e. highly resistant, were considered as uninfected.

As can be deduced from these tables, the 9 hybrids according to the invention are the sole resistant plants, with 100% of the tested plants being uninfected. The tests have been reproduced in different localizations in Australia, for one given hybrid according to the invention: white rust has never been observed for this hybrid, confirming that the resistance to white rust caused by the oomycete *Albugo candida* race 9 Australian variant is indeed a general resistance, which is not strain-dependent.

**Table 12:**

| **Genotype** | Repetition 1 | | | | | | Repetition 2 | | | | | | Repetition 3 | | | | | | Repetition 4 | | | | | | **mean** | SD |
|---|---|---|---|---|---|---|---|---|---|---|---|---|---|---|---|---|---|---|---|---|---|---|---|---|---|---|
| | *pl1* | *pl2* | *pl3* | *pl4* | *pl5* | *pl6* | *pl1* | *pl2* | *pl3* | *pl4* | *pl5* | *pl6* | *pl1* | *pl2* | *pl3* | *pl4* | *pl5* | *pl6* | *pl1* | *pl2* | *pl3* | *pl4* | *pl5* | *pl6* | | |
| **A** | 1 | 3 | 5 | 5 | 5 | 7 | 5 | 5 | 5 | 5 | 5 | 5 | 3 | 5 | 1 | 3 | 5 | | 5 | 3 | 5 | 3 | 5 | 3 | **4.22** | 1.44 |
| **B** | 5 | 5 | 5 | 5 | 7 | 8 | 3 | 5 | | 7 | 1 | 7 | 1 | 1 | 5 | 1 | 3 | 5 | 5 | 1 | 1 | 1 | 1 | 5 | **3.83** | 2.39 |
| **C** | 9 | 9 | 9 | 9 | 9 | 9 | 9 | 9 | 9 | 9 | 9 | 9 | 9 | 9 | 9 | 9 | 9 | 9 | 9 | 9 | 9 | 9 | 9 | 9 | **9.00** | 0.00 |
| **D** | 3 | 1 | 1 | 1 | 1 | 3 | 1 | 1 | 1 | 1 | 1 | 3 | 1 | 1 | 1 | 3 | 1 | 1 | 3 | 1 | 3 | 1 | | 1 | **1.52** | 0.90 |
| **E** | 9 | 9 | 9 | 9 | 9 | 9 | 9 | 9 | 9 | | 9 | 9 | 9 | 9 | 9 | 9 | 9 | 9 | 9 | 9 | 9 | 9 | 9 | 9 | **9.00** | 0.00 |
| **F** | 9 | | 9 | | | | | | | 9 | | 9 | 9 | | | | | 9 | 9 | | | | | 9 | **9.00** | 0.00 |

| | | | | | | | | | | | | | | | | | | | | | | | | | | |
|---|---|---|---|---|---|---|---|---|---|---|---|---|---|---|---|---|---|---|---|---|---|---|---|---|---|---|
| pl = plant SD: Standard deviation | | | | | | | | | | | | | | | | | | | | | | | | | | |

**Table 13: results of field trials conducted in Year N**

| | White Blister Reading | | |
|---|---|---|---|
| **Hybrid name** | **total no. of plants** | **total no. infected** | **% of plants infected** |
| ANACONDA | 17 | 17 | 100,00% |
| AURORA F1 | 20 | 18 | 90,00% |
| AURORA F1 | 21 | 21 | 100,00% |
| AURORA F1 | 18 | 17 | 94,44% |
| AVENGER | 18 | 18 | 100,00% |
| EVEREST | 24 | 22 | 91,67% |
| GREEN MAGIC | 20 | 18 | 90,00% |
| **Inbred line containing the resistance of the invention** | **20** | **0** | **0,00%** |
| JET DOME F1 | 22 | 22 | 100,00% |
| KECHUA | 20 | 18 | 90,00% |
| MONTECO | 18 | 15 | 83,33% |
| PARTHENON | 16 | 16 | 100,00% |
| R1306 F1 | 18 | 16 | 88,89% |
| SPEED DOME F1 | 24 | 22 | 91,67% |
| SPINKS | 9 | 9 | 100,00% |
| SPINKS | 18 | 17 | 94,44% |
| STEEL | 18 | 18 | 100,00% |
| TB 6300 | 22 | 20 | 90,91% |
| TIE MOUNTAIN | 15 | 14 | 93,33% |
| TYSON | 17 | 17 | 100,00% |

**Table 14: results of field trials conducted in Year N+1**

| **Hybrid name** | **Total N° Of Plants:** | **N° Of Plants infected with White Rust:** | **% of plants infected** |
|---|---|---|---|
| GREEN MAGIC | 18 | 18 | 100,00% |
| SPEED DOME | 18 | 18 | 100,00% |
| JET DOME | 18 | 16 | 88,89% |
| NAXOS | 9 | 9 | 100,00% |
| MONACO | 18 | 18 | 100,00% |
| GREEN CANON | 17 | 15 | 88,24% |
| SARASOTA | 6 | 4 | 66,67% |
| CENTENNIAL | 16 | 16 | 100,00% |
| GUERRERO | 13 | 12 | 92,31% |
| ANACONDA | 15 | 7 | 46,67% |
| RUMBA | 17 | 17 | 100,00% |
| PHAROS | 16 | 10 | 62,50% |
| TYSON | 18 | 12 | 66,67% |
| FLORAPACK | 17 | 17 | 100,00% |
| STELL | 16 | 15 | 93,75% |
| EXPO | 14 | 14 | 100,00% |
| 534 | 14 | 11 | 78,57% |
| AURORA F1 | 18 | 17 | 94,44% |
| IRONMAN | 14 | 14 | 100,00% |
| AVENGER | 16 | 15 | 93,75% |
| SPINKS | 16 | 15 | 93,75% |
| HERITAGE | 16 | 16 | 100,00% |
| PARTHENON | 13 | 10 | 76,92% |
| Hybrid H1containing the resistance of the invention | 17 | 0 | 0,00% |
| Other Hybrid H2 containing the resistance of the invention | 14 | 0 | 0,00% |
| Other Hybrid H3 containing the resistance of the invention | 16 | 0 | 0,00% |
| Other Hybrid H4 containing the resistance of the invention | 17 | 0 | 0,00% |
| Other Hybrid H5 containing the resistance of the invention | 15 | 0 | 0,00% |
| Other Hybrid H6 containing the resistance of the invention | 9 | 0 | 0,00% |
| Other Hybrid H7 containing the resistance of the invention | 17 | 0 | 0,00% |
| Other Hybrid H8 containing the resistance of the invention | 16 | 0 | 0,00% |
| Other Hybrid H9 containing the resistance of the invention | 15 | 0 | 0,00% |

## Claims

1. A *Brassica oleracea* var. *italica* broccoli plant resistant to white blister rust caused by the oomycete *Albugo candida* race 9 Australian variant, having in its genome introgressed sequences conferring the resistance,
wherein said introgressed sequences are located on chromosome 4, within the chromosomal region delimited by SNP BO-0108436 (SEQ ID No:5) and SNP BN-0067793 (SEQ ID No:15), preferably within the region delimited by SNP BO-0108436 (SEQ ID No:5) and SNP BO-0108751 (SEQ ID N°11) and are chosen from the introgressed sequences present in the genome of a seed of *B. oleracea* BROCCO-C4, deposited at NCIMB accession number NCIMB-42433, on chromosome 4 and conferring the resistance.

2. A *B. oleracea* plant according to claim 1, wherein the introgressed sequences are homozygously or heterozygously present in the genome of the plant.

3. The plant according to any one of claims 1 to 2, wherein the presence of said introgressed sequences on at least one chromosome 4 and conferring said resistance is **characterized by** at least one marker selected from the group consisting of SNP BO-0108436 (SEQ ID No:5), SNP BO-0108746 (SEQ ID No:6), SNP BO-0108747 (SEQ ID No:7), SNP BO-0108748 (SEQ ID No:8), SNP BO-0108749 (SEQ ID No:9), SNP BO-0108750 (SEQ ID No:10), SNP BO-0108751 (SEQ ID No:11), SNP BO-0108752 (SEQ ID No:12), SNP BO-0108753 (SEQ ID No:13), SNP BN-0067786 (SEQ ID No:14) and SNP BN-0067793 (SEQ ID No:15).

4. The plant according to claim 3, wherein the presence of said introgressed sequences is **characterized by**:
- the presence of allele G of SNP BO-0108436 (SEQ ID No:5); and/or
- the presence of allele C of SNP BO-0108746 (SEQ ID No:6); and/or
- the presence of allele G of SNP BO-0108747 (SEQ ID No:7); and/or
- the presence of allele A of SNP BO-0108748 (SEQ ID No:8); and/or
- the presence of allele C of SNP BO-0108749 (SEQ ID No:9); and/or
- the presence of allele A of SNP BO-0108750 (SEQ ID No:10); and/or
- the presence of allele G of SNP BO-0108751 (SEQ ID No:11); and/or
- the presence of allele C of SNP BO-0108752 (SEQ ID No:12); and/or
- the presence of allele C of SNP BO-0108753 (SEQ ID No:13); and/or
- the presence of allele T of SNP BN-0067786 (SEQ ID No:14); and/or
- the presence of allele G of SNP BN-0067793 (SEQ ID No:15).

5. The plant according to any one of claims 1 to 4, wherein said plant is a progeny of, or is derived from a plant of BROCCO-C4 (NCIMB accession number 42433).

6. A cell of a *B. oleracea* plant according to any one of claims 1 to 5, comprising in its genome introgressed sequences conferring resistance to white blister rust caused by the oomycete *Albugo candida* race 9 Australian variant, wherein the introgressed sequences are chosen from those present in the genome of a plant of the BROCCO-C4 plant (NCIMB accession number 42433) within the chromosomal region delimited by SNP BO-0108436 (SEQ ID No:5) and SNP BN-0067793 (SEQ ID No:15), and preferably between SNP BO-0108436 (SEQ ID No:5) and SNP BO-0108751 (SEQ ID N°11).

7. A plant part, explant, scion, cutting, seed, root, rootstock, pollen, ovule, embryo, siliqua, protoplast, leaf, anther, stem, petiole or head of a *B. oleracea* plant having in its genome introgressed sequences conferring resistance to white blister rust caused by the oomycete *Albugo candida* race 9 Australian variant,
wherein said plant, explants, scion, cutting, seed, root, rootstock, pollen, ovule, embryo, siliqua, protoplast, leaf, anther, stem, petiole or head and/or plant part is obtainable from a plant according to any one of claims 1 to 5 and comprises cells according to claim 6.

8. The cell according to claim 6, which is isolated.

9. A tissue culture of regenerable cells of the plant according to any one of claims 1 to 5, wherein the regenerable cells are derived from embryos, protoplasts, meristematic cells, callus, pollen, leaves, anthers, stems, petioles, roots, root tips, siliqua, seeds, flowers, cotyledons, and/or hypocotyls, and contain in their genome introgressed sequences on chromosome 4 conferring resistance to white blister rust caused by the oomycete *Albugo candida* race 9 Australian variant, wherein said introgressed sequences are chosen from the introgressed sequences present in the genome of a seed of *B. oleracea* BROCCO-C4, deposited at NCIMB accession number NCIMB-42433, on chromosome 4 and conferring the resistance.

10. A method for detecting and/or selecting *B. oleracea* var. italica broccoli plants having introgressed sequences as found in the genome of a plant of BROCCO-C4 (NCIMB accession number 42433), said introgressed sequences conferring resistance to white blister rust caused by the oomycete *Albugo candida* race 9 Australian variant, comprising detection of at least one of the following alleles: allele G of SNP BO-0108436 (SEQ ID No:5), allele C of SNP BO-0108746 (SEQ ID No:6), allele G of SNP BO-0108747 (SEQ ID No:7), allele A of SNP BO-0108748 (SEQ ID No:8), allele C of SNP BO-0108749 (SEQ ID No:9), allele A of SNP BO-0108750 (SEQ ID No:10), allele G of SNP BO-0108751 (SEQ ID No:11), allele C of SNP BO-0108752 (SEQ ID No:12), allele C of SNP BO-0108753 (SEQ ID No:13), allele T of SNP BN-0067786 (SEQ ID No:14), and allele G of SNP BN-0067793 (SEQ ID No:15), in a genetic material sample of the plant to be selected.

11. Use of a *Brassica oleracea* var. *italica* broccoli plant resistant to white blister rust caused by the oomycete *Albugo candida* race 9 Australian variant, having in its genome introgressed sequences conferring the resistance,
for restricting the growth and development of the oomycete *Albugo candida* race 9 Australian variant,
wherein said introgressed sequences are located on chromosome 4, within the chromosomal region delimited by SNP BO-0108436 (SEQ ID No:5) and SNP BN-0067793 (SEQ ID No:15), preferably within the region delimited by SNP BO-0108436 (SEQ ID No:5) and SNP BO-0108751 (SEQ ID N°11), and are chosen from the introgressed sequences present in the genome of a seed of *B. oleracea* BROCCO-C4, deposited at NCIMB accession number NCIMB-42433, on chromosome 4 and conferring the resistance.

12. A method for protecting a field of *B. oleracea* plants, against infection by the oomycete *Albugo candida* race 9 Australian variant, said method comprising growing plants exhibiting resistance to said oomycete, wherein said plants are *Brassica oleracea* var. *italica* broccoli plants, having in their genome introgressed sequences conferring said resistance, wherein said introgressed sequences are located on chromosome 4, within the chromosomal region delimited by SNP BO-0108436 (SEQ ID No:5) and SNP BN-0067793 (SEQ ID No:15), preferably within the region delimited by SNP BO-0108436 (SEQ ID No:5) and SNP BO-0108751 (SEQ ID N°11), and are chosen from the introgressed sequences present in the genome of a seed of *B. oleracea* BROCCO-C4, deposited at NCIMB accession number NCIMB-42433, on chromosome 4 and conferring the resistance.

13. A method for controlling white blister rust, comprising growing plants exhibiting resistance to said oomycete, wherein said plants are *Brassica oleracea* var. *italica* broccoli plants, having in their genome introgressed sequences conferring said resistance, wherein said introgressed sequences are located on chromosome 4, within the chromosomal region delimited by SNP BO-0108436 (SEQ ID No:5) and SNP BN-0067793 (SEQ ID No:15), preferably within the region delimited by SNP BO-0108436 (SEQ ID No:5) and SNP BO-0108751 (SEQ ID N°11), and are chosen from the introgressed sequences present in the genome of a seed of *B. oleracea* BROCCO-C4, deposited at NCIMB accession number NCIMB-42433, on chromosome 4 and conferring the resistance.

## Patentansprüche

1. *Brassica oleracea var. italica* Brokkolipflanze, die gegen Weißen Rost resistent ist, der durch den Oomyceten *Albugo candida* Rasse 9 australische Variante verursacht wird, die in ihrem Genom introgressierte Sequenzen aufweist, die die Resistenz verleihen, wobei die introgressierten Sequenzen auf Chromosom 4 lokalisiert sind, innerhalb der chromosomalen Region, die durch SNP BO-0108436 (SEQ ID Nr.: 5) und SNP BN-0067793 (SEQ ID Nr.: 15) begrenzt wird, vorzugsweise innerhalb der Region, die durch SNP BO-0108436 (SEQ ID Nr.: 5) und SNP BO-0108751 (SEQ ID Nr.: 11) begrenzt wird, und ausgewählt sind aus den introgressierten Sequenzen, die in dem Genom eines Samens von *B. oleracea* BROCCO-C4, hinterlegt unter NCIMB-Zugriffsnummer NCIMB-42433, auf Chromosom 4 vorhanden sind und die Resistenz verleihen.

2. *B. oleracea-*Pflanze nach Anspruch 1, wobei die introgressierten Sequenzen homozygot oder heterozygot in dem Genom der Pflanze vorhanden sind.

3. Pflanze nach einem der Ansprüche 1 bis 2, wobei das Vorhandensein der introgressierten Sequenzen auf mindestens einem Chromosom 4 und Verleihen der Resistenz durch mindestens einen Marker gekennzeichnet ist, der aus der Gruppe ausgewählt ist, die aus SNP BO-0108436 (SEQ ID Nr.: 5), SNP BO-0108746 (SEQ ID Nr.: 6), SNP BO-0108747 (SEQ ID Nr.: 7), SNP BO-0108748 (SEQ ID Nr.: 8), SNP BO-0108749 (SEQ ID Nr.: 9), SNP BO-0108750 (SEQ ID Nr.: 10), SNP BO-0108751 (SEQ ID Nr.: 11), SNP BO-0108752 (SEQ ID Nr.: 12), SNP BO-0108753 (SEQ ID Nr.: 13), SNP BN-0067786 (SEQ ID Nr.: 14) und SNP BN-0067793 (SEQ ID Nr.: 15) besteht.

4. Pflanze nach Anspruch 3, wobei das Vorhandensein der introgressierten Sequenzen **gekennzeichnet ist durch**:
- das Vorhandensein von Allel G von SNP BO-0108436 (SEQ ID Nr.: 5); und/oder
- das Vorhandensein von Allel C von SNP BO-0108746 (SEQ ID Nr.: 6); und/oder
- das Vorhandensein von Allel G von SNP BO-0108747 (SEQ ID Nr.: 7); und/oder
- das Vorhandensein von Allel A von SNP BO-0108748 (SEQ ID Nr.: 8); und/oder
- das Vorhandensein von Allel C von SNP BO-0108749 (SEQ ID Nr.: 9); und/oder
- das Vorhandensein von Allel A von SNP BO-0108750 (SEQ ID Nr.: 10); und/oder
- das Vorhandensein von Allel G von SNP BO-0108751 (SEQ ID Nr.: 11); und/oder
- das Vorhandensein von Allel C von SNP BO-0108752 (SEQ ID Nr.: 12); und/oder
- das Vorhandensein von Allel C von SNP BO-0108753 (SEQ ID Nr.: 13); und/oder
- das Vorhandensein von Allel T von SNP BN-0067786 (SEQ ID Nr.: 14); und/oder
- das Vorhandensein von Allel G von SNP BN-0067793 (SEQ ID Nr.: 15).

5. Pflanze nach einem der Ansprüche 1 bis 4, wobei die Pflanze ein Nachkomme oder abgeleitet ist von einer BROCCO-C4-Pflanze (NCIMB-Hinterlegungsnummer 42433).

6. Zelle einer B. oleracea-Pflanze nach einem der Ansprüche 1 bis 5, die in ihrem Genom introgressierte Sequenzen umfasst, die Resistenz gegen Weißen Rost verleihen, der durch den Oomyceten *Albugo candida* Rasse 9 australische Variante verursacht wird, wobei die introgressierten Sequenzen aus jenen ausgewählt sind, die im Genom einer Pflanze der BROCCO-C4-Pflanze (NCIMB-Zugriffsnummer 42433) innerhalb der chromosomalen Region vorhanden sind, die begrenzt wird durch SNP BO-0108436 (SEQ ID Nr.: 5) und SNP BN-0067793 (SEQ ID Nr.: 15), und vorzugsweise zwischen SNP BO-0108436 (SEQ ID Nr.: 5) und SNP BO-0108751 (SEQ ID Nr.: 11).

7. Pflanzenteil, Explantat, Spross, Steckling, Samen, Wurzel, Wurzelstock, Pollen, Ovulum, Embryo, Siliqua, Protoplast, Blatt, Anthere, Sprossachse, Blattstiel oder Kopf einer *B. oleracea*-Pflanze, die in ihrem Genom introgressierte Sequenzen aufweist, die Resistenz gegen Weißen Rost verleihen, der durch den Oomyceten *Albugo candida* Rasse 9 australische Variante verursacht wird,
wobei die/das/der Pflanze, Explantat, Spross, Steckling, Samen, Wurzel, Wurzelstock, Pollen, Ovulum, Embryo, Siliqua, Protoplast, Blatt, Anthere, Sprossachse, Blattstiel oder Kopf und/oder Pflanzenteil aus einer Pflanze nach einem der Ansprüche 1 bis 5 erhältlich ist und Zellen nach Anspruch 6 umfasst.

8. Zelle nach Anspruch 6, die isoliert ist.

9. Gewebekultur regenerierbarer Zellen der Pflanze nach einem der Ansprüche 1 bis 5, wobei die regenerierbaren Zellen von Embryonen, Protoplasten, meristematischen Zellen, Kallus, Pollen, Blättern, Antheren, Sprossachsen, Blattstielen, Wurzeln, Wurzelspitzen, Siliquen, Samen, Blüten, Kotyledonen und/oder Hypokotylen abgeleitet sind, und in ihrem Genom introgressierte Sequenzen auf Chromosom 4 enthalten, die Resistenz gegen Weißen Rost verleihen, der durch den Oomyceten *Albugo candida* Rasse 9 australische Variante verursacht wird, wobei die introgressierten Sequenzen ausgewählt sind aus den introgressierten Sequenzen, die im Genom eines Samens von *B. oleracea* BROCCO-C4, hinterlegt unter der NCIMB-Zugriffsnummer NCIMB-42433, auf Chromosom 4 vorhanden sind und die Resistenz verleihen.

10. Verfahren zum Detektieren und/oder Selektieren von *B*. *oleracea var. italica* Brokkolipflanzen, die introgressierte Sequenzen aufweisen, wie sie in dem Genom einer Pflanze von BROCCO-C4 (NCIMB-Zugriffsnummer 42433) gefunden werden, wobei die introgressierten Sequenzen Resistenz gegen Weißen Rost verleihen, der durch den Oomyceten *Albugo candida* Rasse 9 australische Variante verursacht wird, umfassend Detektion von mindestens einem der folgenden Allele: Allel G von SNP BO-0108436 (SEQ ID Nr.: 5), Allel C von SNP BO-0108746 (SEQ ID Nr.: 6), Allel G von SNP BO-0108747 (SEQ ID Nr.: 7), Allel A von SNP BO-0108748 (SEQ ID Nr.: 8), Allel C von SNP BO-0108749 (SEQ ID Nr.: 9), Allel A von SNP BO-0108750 (SEQ ID Nr.: 10), Allel G von SNP BO-0108751 (SEQ ID Nr.: 11), Allel C von SNP BO-0108752 (SEQ ID Nr.: 12), Allel C von SNP BO-0108753 (SEQ ID Nr.: 13), Allel T von SNP BN-0067786 (SEQ ID Nr.: 14) und Allel G von SNP BN-0067793 (SEQ ID Nr.: 15) in einer Probe genetischen Materials der auszuwählenden Pflanze.

11. Verwendung einer *Brassica oleracea var. italica* Brokkolipflanze, die gegen Weißen Rost resistent ist, der durch den Oomyceten *Albugo candida* Rasse 9 australische Variante verursacht wird, die in ihrem Genom introgressierte Sequenzen aufweist, die die Resistenz verleihen,
zum Einschränken des Wachstums und der Entwicklung des Oomyceten *Albugo candida* Rasse 9 australische Variante,
wobei die introgressierten Sequenzen auf Chromosom 4 lokalisiert sind, innerhalb der chromosomalen Region, die durch SNP BO-0108436 (SEQ ID Nr.: 5) und SNP BN-0067793 (SEQ ID Nr.: 15) begrenzt wird, vorzugsweise innerhalb der Region, die durch SNP BO-0108436 (SEQ ID Nr.: 5) und SNP BO-0108751 (SEQ ID Nr.: 11) begrenzt wird, und ausgewählt sind aus den introgressierten Sequenzen, die in dem Genom eines Samens von *B. oleracea* BROCCO-C4, hinterlegt unter NCIMB-Zugriffsnummer NCIMB-42433, auf Chromosom 4 vorhanden sind und die Resistenz verleihen.

12. Verfahren zum Schützen eines Feldes von B. oleracea-Pflanzen gegen Infektion mit dem Oomyceten *Albugo candida* Rasse 9 australische Variante, wobei das Verfahren Anbauen von Pflanzen umfasst, die Resistenz gegen den Oomyceten zeigen, wobei die Pflanzen *Brassica oleracea var. italica* Brokkolipflanzen sind, die in ihrem Genom introgressierte Sequenzen aufweisen, die die Resistenz verleihen, wobei die introgressierten Sequenzen auf Chromosom 4 lokalisiert sind, innerhalb der chromosomalen Region, die durch SNP BO-0108436 (SEQ ID Nr.: 5) und SNP BN-0067793 (SEQ ID Nr.: 15) begrenzt wird, vorzugsweise innerhalb der Region, die durch SNP BO-0108436 (SEQ ID Nr.: 5) und SNP BO-0108751 (SEQ ID Nr.: 11) begrenzt wird, und ausgewählt sind aus den introgressierten Sequenzen, die in dem Genom eines Samens von *B. oleracea* BROCCO-C4, hinterlegt unter NCIMB-Zugriffsnummer NCIMB-42433, auf Chromosom 4 vorhanden sind und die Resistenz verleihen.

13. Verfahren zum Bekämpfen von Weißem Rost, umfassend Anbauen von Pflanzen, die Resistenz gegen den Oomyceten zeigen, wobei die Pflanzen *Brassica oleracea* var. *italica* Brokkolipflanzen sind, die in ihrem Genom introgressierte Sequenzen aufweisen, die die Resistenz verleihen, wobei die introgressierten Sequenzen auf Chromosom 4 lokalisiert sind, innerhalb der chromosomalen Region, die durch SNP BO-0108436 (SEQ ID Nr.: 5) und SNP BN-0067793 (SEQ ID Nr.: 15) begrenzt wird, vorzugsweise innerhalb der Region, die durch SNP BO-0108436 (SEQ ID Nr.: 5) und SNP BO-0108751 (SEQ ID Nr.: 11) begrenzt wird, und ausgewählt sind aus den introgressierten Sequenzen, die in dem Genom eines Samens von *B. oleracea* BROCCO-C4, hinterlegt unter NCIMB-Zugriffsnummer NCIMB-42433, auf Chromosom 4 vorhanden sind und die Resistenz verleihen.

## Revendications

1. Plante de brocoli *Brassica oleracea* var.*italica* résistant à la rouille vésiculeuse blanche causée par le variant australien de l'oomycète *Albugo candida* race 9, présentant dans son génome des séquences introgressées conférant la résistance,
dans laquelle lesdites séquences introgressées sont situées sur le chromosome 4, dans la région chromosomique délimitée par le SNP BO-0108436 (SEQ ID No:5) et le SNP BN-0067793 (SEQ ID No:15), de préférence dans la région délimitée par le SNP BO-0108436 (SEQ ID No:5) et le SNP BO-01 08751 (SEQ ID N°11), et sont choisies parmi les séquences introgressées présentes dans le génome d'une graine de *B. oleracea* BROCCO-C4, déposée au NCIMB sous le numéro d'ordre NCIMB-42433, sur le chromosome 4 et conférant la résistance.

2. Plante *B. oleracea* selon la revendication 1, dans laquelle les séquences introgressées sont présentes à l'état homozygote ou hétérozygote dans le génome de la plante.

3. Plante selon l'une quelconque des revendications 1 à 2, dans laquelle la présence desdites séquences introgressées sur au moins un chromosome 4 et conférant ladite résistance, est **caractérisée par** au moins un marqueur sélectionné dans le groupe consistant en SNP BO-0108436 (SEQ ID No:5), SNP BO-0108746 (SEQ ID No:6), SNP BO-0108747 (SEQ ID No:7), SNP BO-0108748 (SEQ ID No:8), SNP BO-0108749 (SEQ ID No:9), SNP BO-0108750 (SEQ ID No:10), SNP BO-0108751 (SEQ ID No:11), SNP BO-0108752 (SEQ ID No:12), SNP BO-0108753 (SEQ ID No:13), SNP BN-0067786 (SEQ ID No:14) et SNP BN-0067793 (SEQ ID No:15).

4. Plante selon la revendication 3, dans laquelle la présence desdites séquences introgressées se **caractérise par** :
- la présence de l'allèle G du SNP BO-0108436 (SEQ ID No:5) ; et/ou
- la présence de l'allèle C du SNP BO-0108746 (SEQ ID No:6) et/ou
- la présence de l'allèle G du SNP BO-0108747 (SEQ ID No:7) ; et/ou
- la présence de l'allèle A du SNP BO-0108748 (SEQ ID No:8) ; et/ou
- la présence de l'allèle C du SNP BO-0108749 (SEQ ID No:9) et/ou
- la présence de l'allèle A du SNP BO-0108750 (SEQ ID No:10) ; et/ou
- la présence de l'allèle G du SNP BO-0108751 (SEQ ID No:11) ; et/ou
- la présence de l'allèle C du SNP BO-0108752 (SEQ ID No:12) et/ou
- la présence de l'allèle C du SNP BO-0108753 (SEQ ID No:13) et/ou
- la présence de l'allèle T du SNP BN-0067786 (SEQ ID No:14) ; et/ou
- la présence de l'allèle G du SNP BN-0067793 (SEQ ID No:15).

5. Plante selon l'une quelconque des revendications 1 à 4, dans laquelle ladite plante est un descendant ou est dérivé d'une plante de BROCCO-C4 (numéro d'ordre NCIMB-42433).

6. Cellule d'une plante *B. oleracea* selon l'une quelconque des revendications 1 à 5, comprenant dans son génome des séquences introgressées conférant une résistance à la rouille vésiculeuse blanche causée par le variant australien de l'oomycète *Albugo candida* race 9, dans laquelle les séquences introgressées sont choisies parmi celles présentes dans le génome d'une plante BROCCO-C4 (numéro d'ordre NCIMB 42433) dans la région chromosomique délimitée par le SNP BO-0108436 (SEQ ID No :5) et le SNP BN-0067793 (SEQ ID No:15), et de préférence entre le SNP BO-0108436 (SEQ ID No:5) et le SNP BO-0108751 (SEQ ID N°11).

7. Partie de plante, explant, scion, bouture, graine, racine, porte-greffe, pollen, ovule, embryon, silique, protoplaste, feuille, anthère, tige, pétiole ou tête d'une plante *B. oleracea* présentant dans son génome des séquences introgressées conférant une résistance à la rouille vésiculeuse blanche causée par le variant australien de l'oomycète *Albugo candida* race 9,
dans laquelle ladite plante, explant, scion, bouture, semence, fruit, racine, porte-greffe, pollen, ovule, embryon, silique, protoplaste, feuille, anthère, tige, pétiole ou tête et/ou partie de plante est susceptible d'être obtenu à partir d'une plante selon l'une quelconque des revendications 1 à 5, et comprend des cellules selon la revendication 6.

8. Cellule selon la revendication 6, qui est isolée.

9. Culture tissulaire de cellules régénérables de la plante selon l'une quelconque des revendications 1 à 5, dans laquelle les cellules régénérables sont dérivées d'embryons, de protoplastes, de cellules méristématiques, de cals, de pollen, de feuilles, d'anthères, de tiges, de pétioles, de racines, d'extrémités de racines, de silique, de graines, de fleurs, de cotylédons et/ou d'hypocotyles, et contiennent dans leur génome des séquences introgressées sur le chromosome 4 conférant une résistance à la rouille vésiculeuse blanche causée par le variant australien de l'oomycète *Albugo candida* race 9, lesdites séquences introgressées étant choisies parmi les séquences introgressées présentes dans le génome d'une graine de *B. oleracea* BROCCO-C4, déposée au NCIMB sous le numéro d'ordre NCIMB-42433, sur le chromosome 4 et conférant la résistance.

10. Procédé de détection et/ou de sélection de plants de brocoli *B. oleracea* var. italica présentant des séquences introgressées telles que trouvées dans le génome d'une plante de BROCCO-C4 (numéro d'ordre NCIMB 42433), lesdites séquences introgressées conférant une résistance à la rouille vésiculeuse blanche causée par le variant australien de l'oomycète *Albugo candida* race 9, comprenant la détection d'au moins un des allèles suivants : allèle G du SNP BO-0108436 (SEQ ID No:5), allèle C du SNP BO-0108746 (SEQ ID No:6), allèle G du SNP BO-0108747 (SEQ ID No :6), allèle G du SNP BO-0108747 (SEQ ID No:7), allèle A du SNP BO-0108748 (SEQ ID No:8), allèle C du SNP BO-0108749 (SEQ ID No:9), allèle A du SNP BO-0108750 (SEQ ID No:10), allèle G du SNP BO-0108751 (SEQ ID No :11), l'allèle C du SNP BO-0108752 (SEQ ID No:12), l'allèle C du SNP BO-0108753 (SEQ ID No:13), l'allèle T du SNP BN-0067786 (SEQ ID No:14), et l'allèle G du SNP BN-0067793 (SEQ ID No:15), dans un échantillon de matériel génétique de la plante à sélectionner.

11. Utilisation d'un brocoli *Brassica oleracea* var*.italica* résistant à la rouille vésiculeuse blanche causée par le variant australien de l'oomycète *Albugo candida* race 9, présentant dans son génome des séquences introgressées conférant la résistance,
pour limiter la croissance et le développement du variant australien de l'oomycète *Albugo candida* race 9,
dans laquelle lesdites séquences introgressées sont situées sur le chromosome 4, dans la région chromosomique délimitée par le SNP BO-0108436 (SEQ ID No:5) et le SNP BN-0067793 (SEQ ID No:15), de préférence dans la région délimitée par le SNP BO-0108436 (SEQ ID No:5) et le SNP BO-01 08751 (SEQ ID N°11), et sont choisies parmi les séquences introgressées présentes dans le génome d'une graine de *B. oleracea* BROCCO-C4, déposée au NCIMB sous le numéro d'ordre NCIMB-42433, sur le chromosome 4 et conférant la résistance.

12. Procédé de protection d'un champ de plantes *B. oleracea* contre l'infection par le variant australien de l'oomycète *Albugo candida* race 9, ledit procédé consistant à cultiver des plantes présentant une résistance audit oomycète, dans lequel lesdites plantes sont des plantes de *Brassica oleracea* var. *italica,* présentant dans leur génome des séquences introgressées conférant ladite résistance, dans lequel lesdites séquences introgressées sont situées sur le chromosome 4, dans la région chromosomique délimitée par le SNP BO-0108436 (SEQ ID No:5) et le SNP BN-0067793 (SEQ ID No:15), de préférence dans la région délimitée par le SNP BO-0108436 (SEQ ID No:5) et le SNP BO-0108751 (SEQ ID N°11), et sont choisies parmi les séquences introgressées présentes dans le génome d'une graine de *B. oleracea* BROCCO-C4, déposée au NCIMB sous le numéro d'ordre NCIMB-42433, sur le chromosome 4 et conférant la résistance.

13. Procédé de lutte contre la rouille vésiculeuse blanche, comprenant la culture de plantes présentant une résistance audit oomycète, dans lequel lesdites plantes sont des plantes de *Brassica oleracea* var. *italica,* présentant dans leur génome des séquences introgressées conférant ladite résistance, dans lequel lesdites séquences introgressées sont situées sur le chromosome 4, dans la région chromosomique délimitée par les SNP BO-0108436 (SEQ ID No:5) et SNP BN-0067793 (SEQ ID No:15), de préférence dans la région délimitée par le SNP BO-0108436 (SEQ ID No:5) et le SNP BO-0108751 (SEQ ID N°11), et sont choisies parmi les séquences introgressées présentes dans le génome d'une graine de *B. oleracea* BROCCO-C4, déposée au NCIMB sous le numéro d'ordre NCIMB-42433, sur le chromosome 4 et conférant la résistance.
